(19) Europäisches Patentamt
European Patent Office
Office européen des brevets

(11) EP 3 586 822 A1

(12) **EUROPEAN PATENT APPLICATION**
published in accordance with Art. 153(4) EPC

(43) Date of publication:
**01.01.2020 Bulletin 2020/01**

(21) Application number: **18758379.4**

(22) Date of filing: **21.02.2018**

(51) Int Cl.:
***A61K 8/81*** *(2006.01)* ***A61K 8/19*** *(2006.01)*
***A61K 8/31*** *(2006.01)* ***A61K 8/34*** *(2006.01)*
***A61K 8/36*** *(2006.01)* ***A61K 8/37*** *(2006.01)*
***A61K 8/891*** *(2006.01)* ***A61K 8/92*** *(2006.01)*
***A61Q 1/00*** *(2006.01)*

(86) International application number:
**PCT/JP2018/006126**

(87) International publication number:
**WO 2018/155470 (30.08.2018 Gazette 2018/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **22.02.2017 JP 2017031253**

(71) Applicant: **Nisshinbo Holdings Inc.**
**Tokyo 103-8650 (JP)**

(72) Inventors:
- **HAYAKAWA Kazutoshi**
**Chiba-city**
**Chiba 267-0056 (JP)**
- **HASHIBA Toshifumi**
**Chiba-city**
**Chiba 267-0056 (JP)**

(74) Representative: **Mewburn Ellis LLP**
**City Tower**
**40 Basinghall Street**
**London EC2V 5DE (GB)**

(54) **SKIN COSMETIC**

(57) Provided is a skin cosmetic that includes: (A) 0.1-40 mass% of polymer particles that include prolate-spheroidal polymer particles A that have no flat surfaces, that (1), in a two-dimensional projection obtained by shining light from a direction that is orthogonal to a longitudinal-axis direction, have an average ($L^1_{AV}$) major axis ($L^1$) of 3-60 μm, that (2), in the two-dimensional projection obtained by shining light from the direction that is orthogonal to the longitudinal-axis direction, have an average ($D^1_{AV}$) minor axis ($D^1$) of 0.5-30 μm, and that (3) have an average ($P^1_{AV}$) aspect ratio ($L^1/D^1$), as calculated from the major axes ($L^1$) and the minor axes ($D^1$), of 1.5-30; and 60-99.9 mass% of other components that include (B) inorganic particles and (C) an oily component.

**FIG.1**

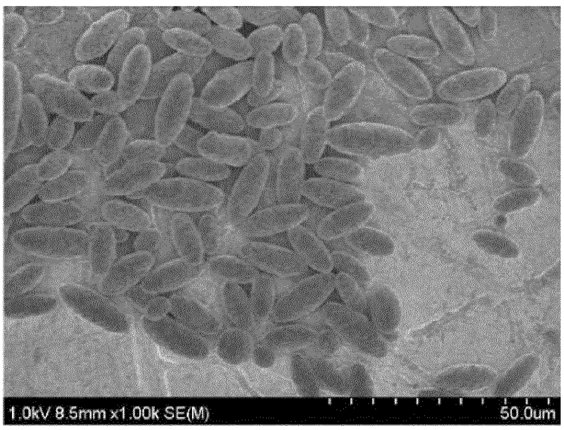


EP 3 586 822 A1

## Description

### TECHNICAL FIELD

The present invention relates to a skin cosmetic.

### BACKGROUND ART

Micron-size polymer particles and inorganic particles are used as fillers and specimens in various fields such as electronic/electrical materials, optical materials, paints, inks, construction materials, biological/pharmaceutical materials, and cosmetics. Among them, particles having an irregular shape different from a spherical shape can exhibit various properties different from those of spherical particles in terms of optical properties, feelings and the like, and thus, have been actively developed in recent years, and the application development of the particles have been carried out on a daily basis.

The present inventors have also developed ellipsoidal or needle-shaped polymer particles having a high aspect ratio so far, found a variety of particles superior to conventional spherical particles in terms of properties including hiding properties, light diffusibility, and feelings, and been promoting the development for cosmetic uses (Patent Documents: 1 to 5). However, in the art, novel polymer particles and uses thereof are being developed to further improve the properties.

In general, in cosmetic uses, spherical polymer particles are frequently used as extenders and feeling agents. These are also very useful from the viewpoint of imparting slipperiness, lightness and the like. However, though the improvement of adhesion and long lasting performance are required for skin cosmetics such as foundations and point makeup cosmetics, spherical particles tend to peel off the applied surface over time, and the amount of application is limited.

### PRIOR ART DOCUMENTS

### PATENT DOCUMENTS

Patent Document 1: JP-A 2009-235353
Patent Document 2: JP-A 2009-235355
Patent Document 3: JP-A 2015-93973
Patent Document 4: WO 2016/181876
Patent Document 5: WO 2016/181878

### SUMMARY OF INVENTION

### TECHNICAL PROBLEM

The present invention has been made under the above circumstances, and an object thereof is to provide a skin cosmetic that has high adhesion and is excellent in optical properties such as hiding properties, light diffusibility, and protection against ultraviolet ray (UV), feelings, and fluidity.

### SOLUTION TO PROBLEM

As a result of intensive studies to solve the above-mentioned problems, the present inventors have found that prolate-spheroidal polymer particles have high adhesion and unique properties different from those of conventional polymer particles in terms of light diffusibility, protection against UV, feelings, fluidity and the like, and are useful for a skin cosmetic, thereby completing the present invention.

That is, the present invention provides the following skin cosmetic.

1. A skin cosmetic including:

(A) 0.1 to 40% by weight of polymer particles including prolate-spheroidal polymer particles A that satisfy (1) to (3) below:

(1) an average ($L^1Av$) of a long diameter ($L^1$) of a two-dimensional projection obtained by radiating light from a direction orthogonal to a major axis direction of 3 to 60 μm;
(2) an average ($D^1_{AV}$) of a short diameter ($D^1$) of a two-dimensional projection obtained by radiating light from a direction orthogonal to a major axis direction of 0.5 to 30 μm; and
(3) an average ($P^1_{AV}$) of an aspect ratio ($L^1/D^1$) calculated from the long diameter ($L^1$) and the short diameter ($D^1$) of 1.5 to 30,

and have no flat surface; and
60 to 99.9% by weight of other components including (B) inorganic particles and an (C) oily component.

2. The skin cosmetic according to 1, wherein the prolate-spheroidal polymer particles A further satisfy (4).
(4) a softening temperature of 120 to 180°C.
3. The skin cosmetic according to 1 or 2, wherein the prolate-spheroidal polymer particles A have, at least on a surface or in a surface layer of the prolate-spheroidal polymer particles A, fine irregularities formed by binding or containing fine particles that are composed of a component same as a component of the prolate-spheroidal polymer particles A and satisfy (5) below:
(5) a particle size ($SP^1$) of the fine particles which are attached or contained on the surface or in the surface layer is $1/1000 \times D^1_{AV} \leq SP^1 \leq 1/2 \times D^1_{AV}$.
4. The skin cosmetic according to any one of 1 to 3, wherein the prolate-spheroidal polymer particles A include a styrene polymer or a (meth)acrylic polymer.
5. The skin cosmetic according to any one of 1 to 4, wherein the prolate-spheroidal polymer particles A have a bulk density of 0.1 to 0.7 g/mL.
6. The skin cosmetic according to any one of 1 to 5, wherein the prolate-spheroidal polymer particles A have a volume mean particle size ($MV^1$) of 0.1 to 50 μm and satisfy a dispersion range ($SD^1$) of 1 to 30.
7. The skin cosmetic according to any one of 1 to 6, wherein the polymer particles (A) include two or more types of the prolate-spheroidal polymer particles A.
8. The skin cosmetic according to any one of 1 to 7, wherein the polymer particles (A) further include one or more types of polymer particles B having a shape different from a shape of the prolate-spheroidal polymer particles A.
9. The skin cosmetic according to 8, wherein the polymer particles B are flat ellipsoidal polymer particles that have a front view, a plan view, and a side view of projections according to third angle projection that are all ellipses, and that satisfy (6) to (8) below:

(6) an average ($L^2_{AV}$) of a long diameter ($L^2$) of a flat part of $0.13 \leq L^2_{AV} \leq 500$ μm;
(7) an average ($D^2_{AV}$) of a short diameter ($D^2$) of a flat part of $0.1 \leq D^2_{AV} \leq 250$ μm; and
(8) an average ($P^2_{AV}$) of an aspect ratio ($L^2/D^2$) calculated from the long diameter ($L^2$) and the short diameter ($D^2$) of $1.3 < P^2_{AV} \leq 50$.

10. The skin cosmetic according to 8 or 9, having a compounding ratio of the prolate-spheroidal polymer particles A to the polymer particles B of 99:1 to 10:90 in terms of weight ratio.
11. The skin cosmetic according to any one of 7 to 10, wherein the polymer particles (A) are polymer particles in which two or more types of polymer particles are combined so that the polymer particles in which two or more types of polymer particles are combined satisfy at least one of (12), (13), and (14) below:

(12) a difference between a maximum and a minimum of an average of an aspect ratio of two or more;
(13) a difference between a maximum and a minimum of a volume mean particle size of 3 μm or more; and
(14) appearance of two or more peaks in particle size distribution measurement.

12. The skin cosmetic according to any one of 7 to 11, wherein the polymer particles in which two or more types of polymer particles are combined have a difference between a volume mean particle size (MV) and a number mean particle size (MN) of 1.5 μm or more.
13. The skin cosmetic according to any one of 1 to 11, wherein the inorganic particles (B) have a plate-like shape or a scaly shape.
14. The skin cosmetic according to any one of 1 to 12, wherein the oily component (C) is at least one selected from a higher alcohol, a higher fatty acid, a fat and oil, a wax, a hydrocarbon, a silicone, and a fatty acid ester.

## ADVANTAGEOUS EFFECTS OF INVENTION

The skin cosmetic of the present invention includes prolate-spheroidal polymer particles A as a necessary component, and thus, from the viewpoint of optical properties, slipperiness, adhesiveness and the like resulting from their shapes, are useful as a makeup cosmetic such as an eyeliner, an eyebrow pencil, an eye shadow, a lipstick, a rouge, a foundation, a concealer, a face powder, a base makeup cosmetic such as a makeup base, and a point makeup cosmetic. Specifically, optical properties thereof make the skin look brighter, and the gradation effect can improve the covering performance. A UV diffusion effect can be also added. Further, due to the slipperiness (fluidity) unique to the prolate-spheroidal shape, the present invention is excellent in extension on the skin, and further finely fills grooves of texture, which can make wrinkles and pores less noticeable. Due to the high adhesiveness, a high effect can be obtained with even a small amount, and non-conventional cosmetic effects can be obtained. A finish feeling unique to the polymer component can be added.

Further, the strength of shaped products such as a pressed powder and a lipstick can be improved, and a cosmetic that is useful also from the viewpoint of quality can be obtained. The present invention can be used in many cosmetic uses by adjusting the shape of polymer particles or using two or more types of polymer particles having different shapes in combination according to the balance of product properties, product concept and the like.

## BRIEF DESCRIPTION OF DRAWINGS

[FIG. 1] FIG. 1 is a scanning electron microscope (SEM) photograph (1,000×) of the prolate-spheroidal polymer particles A1 obtained in Production Example 1.
[FIG. 2] FIG. 2 is a SEM photograph (1,000×) of the prolate-spheroidal polymer particles A1 obtained in Production Example 1 taken from a direction 45° to the direction from which the photograph in FIG. 1 was taken.
[FIG. 3] FIG. 3 is a SEM photograph (1,000×) of the prolate-spheroidal polymer particles A2 obtained in Production Example 2.
[FIG. 4] FIG. 4 is a SEM photograph (1,000×) of the prolate-spheroidal polymer particles A2 obtained in Production Example 2 taken from a direction 45° to the direction from which the photograph in FIG. 3 was taken.
[FIG. 5] FIG. 5 is a SEM photograph (1,000×) of the prolate-spheroidal polymer particles A3 obtained in Production Example 3.
[FIG. 6] FIG. 6 is a SEM photograph (1,000×) of the prolate-spheroidal polymer particles A3 obtained in Production Example 3 taken from a direction 45° to the direction from which the photograph in FIG. 5 was taken.
[FIG. 7] FIG. 7 is a SEM photograph (1,000×) of the prolate-spheroidal polymer particles A4 obtained in Production Example 4.
[FIG. 8] FIG. 8 is a SEM photograph (1,000×) of the prolate-spheroidal polymer particles A4 obtained in Production Example 4 taken from a direction 45° to the direction from which the photograph in FIG. 7 was taken.
[FIG. 9] FIG. 9 is a SEM photograph (1,000×) of the prolate-spheroidal polymer particles A5 obtained in Production Example 5.
[FIG. 10] FIG. 10 is a SEM photograph (1,000×) of the prolate-spheroidal polymer particles A5 obtained in Production Example 5 taken from a direction 45° to the direction from which the photograph in FIG. 9 was taken.
[FIG. 11] FIG. 11 is a SEM photograph (2,000×) of the prolate-spheroidal polymer particles A6 obtained in Production Example 6.
[FIG. 12] FIG. 12 is a SEM photograph (2,000×) of the prolate-spheroidal polymer particles A6 obtained in Production Example 6 taken from a direction 45° to the direction from which the photograph in FIG. 11 was taken.
[FIG. 13] FIG. 13 is a diagram showing the long diameter ($L^2$), the short diameter ($D^2$), and the thickness ($T^2$) of flat ellipsoidal polymer particles.

## DESCRIPTION OF EMBODIMENTS

[Skin cosmetic]

The skin cosmetic of the present invention includes polymer particles (A) including prolate-spheroidal polymer particles A that have no flat surface, and inorganic particles (B) and an oily component (C) as other components. In the present invention, "prolate-spheroidal" refers to, in addition to a shape of a long sphere that is a prolate spheroid obtained by rotating an ellipse around its major axis as a rotation axis, all shapes called an approximate prolate spheroid, a spindle, a needle, or a bar.

[(A) Polymer particles]

[Prolate-spheroidal polymer particles A]

The prolate-spheroidal polymer particles A included in component (A) satisfy (1) to (3) below:

(1) an average ($L^1_{AV}$) of a long diameter ($L^1$) of a two-dimensional projection obtained by radiating light from a direction orthogonal to a major axis direction of 3 to 60 μm;
(2) an average ($D^1_{AV}$) of a short diameter ($D^1$) of a two-dimensional projection obtained by radiating light from a direction orthogonal to a major axis direction of 0.5 to 30 μm; and
(3) an average ($P^1_{AV}$) of an aspect ratio ($L^1/D^1$) calculated from the long diameter ($L^1$) and the short diameter ($D^1$) of 1.5 to 30,

and have no flat surface.
$L^1_{AV}$ of the prolate-spheroidal polymer particles A is 3 to 60 μm. $L^1_{AV}$ is preferably 5 to 55 μm, more preferably 8 to 50 μm, and further preferably 10 to 50 μm in consideration of the fine finish of makeup. If $L^1_{AV}$ is more than 60 μm, properties of the particles become similar to those of general-purpose fibers, and the superiority is lost. With the reduction of specific surface area per unit, optical properties such as light scattering tend to be significantly deteriorated. If $L^1_{Av}$ is less than 3 μm, the short diameter also becomes thin, and thus the optical properties may be deteriorated and the strength may be reduced.
$D^1_{AV}$ of the prolate-spheroidal polymer particles A is 0.5 to 30 μm. $D^1_{AV}$ is preferably 0.8 to 25 μm, more preferably 1 to 20 μm, and further preferably 1 to 15 μm in consideration of the fine finish of makeup. If $D^1_{AV}$ is more than 30 μm, properties of the particles become similar to those of general-purpose spherical particles, and with the reduction of specific surface area per unit, the effect of optical properties tend to be significantly deteriorated. If $D^1_{AV}$ is less than 0.5 μm, the optical properties may be deteriorated and the strength may be reduced.
$P^1_{AV}$ of the prolate-spheroidal polymer particles A is 1.5 to 30. $P^1_{AV}$ is preferably 1.8 to 25, more preferably 2 to 20, still more preferably 2.2 to 20, and further preferably 2.5 to 18 in consideration of the fine finish of makeup. If optical properties such as light diffusibility are emphasized, it is particularly preferably 3 to 18. If $P^1_{AV}$ is more than 30, the obtained particles tend to be oriented, and optical properties such as light scattering and light reflectivity may not be stably obtained. If $P^1_{AV}$ is less than 1.5, the effect of optical properties becomes similar to those of spherical particles of the same component and the superiority is lost.
The prolate-spheroidal polymer particles A preferably have a volume mean particle size ($MV^1$) of 0.1 to 50 μm, more preferably 0.5 to 40 μm, further preferably 1 to 30 μm, and most preferably 2 to 20 μm. Further, the prolate-spheroidal polymer particles A are preferably particles whose distribution range is adjusted so that the dispersion range ($SD^1$) in the particle size distribution of 1 to 30 is satisfied. $SD^1$ is more preferably 1.5 to 20, further preferably 2 to 15, and most preferably 2.5 to 12. If $MV^1$ and $SD^1$ are in the above-mentioned range, the properties of the prolate-spheroidal polymer particles can be efficiently utilized. In the present invention, the volume mean particle size is a measurement obtained by a laser diffraction/scattering method, and means an average particle size when the volume is converted into a sphere. The dispersion range (SD) is a standard deviation calculated from the following formula that is an index of the distribution range of the particle size distribution.

$$SD = (D_{84} - D_{16})/2$$

$D_{84}$: Particle size (μm) at the point of 84% in the cumulative curve
$D_{16}$: Particle size (μm) at the point of 16% in the cumulative curve

The prolate-spheroidal polymer particles A preferably have a softening temperature of 120 to 180°C, more preferably 125 to 175°C, and still more preferably 130 to 170°C. If the softening temperature is in the above-mentioned range, the feeling is good and the particles can be applied to a wide range of skin cosmetics. In the present invention, the softening temperature is a temperature of an endothermic peak in a DSC curve measured with a differential scanning calorimeter.
The prolate-spheroidal polymer particles A are preferably particles having a characteristic that makes the specific surface area relatively large, such as particles having fine irregularities on its surface and porous particles. In particular, the prolate-spheroidal polymer particles A are particles that have, on at least a surface or in a surface layer, fine irregularities formed by binding or containing fine particles that are composed of a component same as a component of the prolate-spheroidal polymer particles A and satisfy (5) below:
(5) a particle size ($SP^1$) of $1/1000 \times D_{AV}{}^1 \leq SP^1 \leq 1/2 \times D^1_{AV}$.

If (5) above is satisfied, light scattering properties are improved, and the properties of hiding wrinkles and pores and the soft focus properties can be improved.

The fine particles more preferably satisfy (5') below:

(5') a particle size ($SP^1$) of $1/1000 \times D_{AV}^1 \leq SP^1 \leq 1/4 \times D^1_{AV}$.

The prolate-spheroidal polymer particles A preferably have a bulk density of 0.1 to 0.7 g/mL, more preferably 0.15 to 0.65 g/mL, and still more preferably 0.2 to 0.6 g/mL. If the prolate-spheroidal polymer particles A have a bulk density with the above-mentioned range, voluminous feeling can be obtained.

The prolate-spheroidal polymer particles A are preferably particles having water absorption or oil absorption of 50 mL or more per 100 g of particles and having affinity to at least one (or both) of water and oil. The water absorption or the oil absorption is preferably 60 mL or more per 100 g of particles, further preferably 80 mL or more per 100 g of particles, and most preferably 100 mL or more per 100 g.

The polymer component of the prolate-spheroidal polymer particles A is not particularly limited as long as its shape is maintained. However, in consideration of the safety of the cosmetic, components generally used in cosmetic uses are preferable, such as a styrene polymer, a (meth)acrylic polymer, a polyolefin polymer, a polydiene polymer, a carboxylic acid vinyl ester polymer, a poly-N-vinyl compound polymer, a polyamide polymer, a polyester polymer, a silicone polymer, and a polyurethane polymer. These polymers can be crosslinked. These polymers can be homopolymers or copolymers. For example, the "styrene polymer" is a polymer having a constitutional unit obtained from a styrene unsaturated monomer as a main constitutional unit, and includes, in addition to a homopolymer of a styrene unsaturated monomer, a copolymer of different styrene unsaturated monomers, and a copolymer of a styrene unsaturated monomer and other monomers. The polymer component of the prolate-spheroidal polymer particles A is preferably a styrene polymer or a (meth)acrylic polymer from the viewpoint of stable and efficient obtainment in a relatively low temperature range, and industrial practicability, and cost.

Examples of the styrene polymer include a (co)polymer obtained from a styrene monomer such as polystyrene, a styrene-(meth)acrylic acid copolymer, a styrene-(meth)acrylate ester copolymer, an acrylonitrile-styrene copolymer, an acrylonitrile-chlorinated polyethylene-styrene copolymer, a styrene-maleic anhydride copolymer or a modified product thereof, a styrene-butadiene block copolymer (SBR), a styrene-butadiene-styrene block copolymer (SBS), a hydrogenated styrene-butadiene-styrene block copolymer (SEBS), a styrene-isoprene block copolymer (SIR), a styrene-isoprene-styrene block copolymer (SIS), a copolymer of styrene and olefin or conjugated dienes, such as a hydrogenated styrene-isoprene-styrene block copolymer (SEPS).

Examples of the (meth)acrylic polymer include a (meth)acrylic acid (co)polymer, a (meth)acrylate ester (co)polymer, a (meth)acrylic acid-(meth)acrylate ester copolymer, a carboxylic acid vinyl ester-(meth)acrylic acid copolymer, a carboxylic acid vinyl ester-(meth)acrylate ester copolymer, an olefin-(meth)acrylic acid copolymer such as an ethylene-acrylic acid copolymer, an olefin-(meth)acrylate ester copolymer such as an ethylene-acrylate ester copolymer, a N-vinyl compound-(meth)acrylic acid copolymer, a N-vinyl compound-(meth)acrylate ester copolymer, a conjugated diene-(meth)acrylic acid copolymer, and a conjugated diene-(meth)acrylate ester copolymer.

Examples of the polyolefin polymer include a polyolefin, a polyfluorinated olefin, a copolymer of an olefin and/or a fluorinated polyolefin, and an olefin-conjugated diene copolymer. Examples of the polydiene polymer include a (co) polymer of a conjugated diene.

Examples of the carboxylic acid vinyl ester polymer include a (co)polymer of a carboxylic acid vinyl ester, an olefin-carboxylic acid vinyl ester copolymer such as an ethylene-vinyl acetate copolymer, a carboxylic acid vinyl ester-conjugated diene copolymer.

Examples of the poly-N-vinyl compound polymer include a (co)polymer of an N-vinyl compound, a copolymer of an olefin-N-vinyl compound, and a copolymer of a conjugated diene-N-vinyl compound.

Examples of the polyamide polymer include polyamide polymers obtained by polycondensation of dicarboxylic acids such as adipic acid, heptane dicarboxylic acid, octane dicarboxylic acid, nonane dicarboxylic acid, undecane dicarboxylic acid, and dodecane dicarboxylic acid with diamines such as tetramethylenediamine, hexamethylenediamine, octamethylenediamine, nonamethylenediamine, undecamethylenediamine, and dodecamethylenediamine. Other examples include polyamide polymers obtained by ring-opening polymerization of lactams such as $\alpha$-pyrrolidone, $\varepsilon$-caprolactam, $\omega$-laurolactam, and $\varepsilon$-enantholactam. Specific examples include nylon-6, nylon-11, nylon-12, nylon-6,6, and nylon-6,T.

Examples of the polyester polymer include polyester polymers containing terephthalic acid or dimethyl terephthalate as the main acid component and at least one alkylene glycol selected from ethylene glycol, diethylene glycol, trimethylene glycol, and butylene glycol as the main glycol component, and polylactic acid. Specific examples include polyethylene terephthalate, polyethylene naphthalate, polybutylene terephthalate, polybutylene naphthalate, polytrimethylene terephthalate, polycyclohexylene dimethylene terephthalate, polycyclohexylene dimethylene naphthalate, and polylactic acid.

The silicone polymer is not particularly limited as long as it contains a silicon-silicon bond, a silicon-carbon bond, a siloxane bond, or a silicon-nitrogen bond in the molecular chain. Specific examples include polysiloxane, polycarbosilane, and polysilazane.

Examples of the polyurethane polymer include a polyurethane resin obtained by polymerization of a polyol and a polyisocyanate. In this case, examples of the polyol include ethylene glycol, diethylene glycol, propylene glycol, dipropylene glycol, glycerin, 1,1,1-trimethylolpropane, 1,2,5-hexanetriol, 1,3-butanediol, 1,4-butanediol, 4,4'-dihydroxyphenylpropane, 4,4'-dihydroxyphenylmethane, and pentaerythritol. Examples of the polyisocyanate include 4-tolylene diisocyanate, 2,6-tolylene diisocyanate, 4,4'-diphenylmethane diisocyanate, 2,4'-diphenylmethane diisocyanate, p-phenylene diisocyanate, isophorone diisocyanate, and xylylene diisocyanate.

The molecular weight of the polymer that constitutes the particles is not particularly limited. However, it is usually about 1,000 to 3,000,000 in weight average molecular weight. In the present invention, the weight average molecular weight is a measurement in terms of polystyrene obtained by gel permeation chromatography.

The prolate-spheroidal polymer particles A can be used singly or in combination of two or more types. However, it is preferable that the prolate-spheroidal polymer particles A be used in combination of two or more types, because a synergistic effect can be obtained according to the properties of each type of prolate-spheroidal polymer particles if two or more types are used in combination.

The prolate-spheroidal polymer particles A can be produced according to the methods described in Patent Documents 1 to 5 and the like. In particular, those produced according to the methods described in Patent Documents 3 to 5 are preferable.

Specifically, those are preferable that are produced by a method including the step of heating a synthesis solution containing water, a mixed solvent of a hydrophilic organic solvent and a hydrophobic organic solvent, a polymer stabilizer, a polymerization initiator, and an unsaturated monomer to perform solution polymerization. Those are further preferable that are produced by a method including the step of adjusting the pH of the synthesis solution to 5 or less or 9 or more at least after the start of heating to perform solution polymerization.

Specific examples of the solution polymerization method include a suspension polymerization method, an emulsion polymerization method, a dispersion polymerization method, a seed polymerization method, and a complex method equivalent thereto.

The suspension polymerization method is a method in which a monomer, a polymerization initiator that is soluble in the monomer and the like are mechanically stirred in a medium in which they are insoluble, and the polymerization reaction is allowed to proceed in a suspended state to precipitate or produce polymer particles.

The emulsion polymerization method is a method in which a medium such as water, a monomer that is insoluble in this medium, an emulsifier (a surfactant) and the like are mixed, and at the same time, a polymerization initiator that is soluble in the medium is added to the mixture to allow the polymerization reaction to proceed, thereby polymer particles are precipitated or produced.

The dispersion polymerization method is a method in which polymerization reaction is allowed to proceed in a homogeneous solution in which a monomer, an initiator, a dispersion stabilizer and the like are dissolved in a liquid medium in which a monomer is soluble, but becomes insoluble as the monomer polymerizes, thereby polymer particles are precipitated or produced.

The seed polymerization method is a polymerization method in which other particles that serve as cores are added in advance on the polymerization reaction, and the polymerization reaction is performed on surfaces of the particles.

The prolate-spheroidal polymer particles A can be obtained by these various solution polymerizations. However, the prolate-spheroidal polymer particles A are more preferably obtained by suspension polymerization, emulsion polymerization, dispersion polymerization, or a combination thereof. According to these methods, the step of preparing seed particles, which is necessary in seed polymerization, can be omitted.

Though the adjustment of pH is performed at least after the start of heating, it can be performed before the start of heating. After adjusting the pH of the synthesis solution to 5 or less or 9 or more, it is preferable to keep the pH at 5 or less or 9 or more until the reaction is completed.

The pH of the synthesis solution is preferably 0 to 5 or 9 to 14, more preferably 0 to 4 or 10 to 14, further preferably 0 to 3 or 11 to 14, and most preferably 0 to 2 or 12 to 14. As described above, if the pH is shifted to the acid or alkaline side, radical polymerization proceeds stably to form prolate-spheroidal polymer particles. Shapes having unique sizes, aspect ratios and the like can be easily controlled, thus, impurities such as aggregates and stuck particles are reduced, and particles can be stably obtained. The pH is preferably 0 to 5 because the polymerization reaction proceeds more stably if the reaction is allowed to proceed at pH shifted to the acid side.

Examples of the method of adjusting pH include a method in which for example, after heating is started, a pH adjuster is gradually dropped into the synthesis solution to change the pH to the acid or alkali side. Alternatively, if a persulfate described later is used as a polymerization initiator, it is decomposed during the polymerization reaction to generate an acid, and the pH is lowered gradually. In this case, the pH adjuster need not be added.

Examples of the pH adjuster include acids such as citric acid, tartaric acid, lactic acid, glycolic acid, hydrochloric acid, nitric acid, sodium citrate, sodium lactate, succinic acid, acetic acid, sodium acetate, fumaric acid, sulfuric acid, malic acid, and phosphoric acid, and alkalis such as sodium hydroxide, potassium hydroxide, calcium hydroxide, magnesium hydroxide, sodium carbonate, potassium carbonate, calcium carbonate, ammonium carbonate, ammonia, morpholine, triethanolamine, diethanolamine, dimethylamine, diethylamine, trimethylamine, and triethylamine.

If solution polymerization is performed with the pH of the synthesis solution adjusted to 5 or less or 9 or more, the growth inside or on the surface of the particle is promoted while forming an elliptic framework of the object, and thus, monodisperse prolate-spheroidal polymer particles can be obtained stably with less aggregates and impurities.

In the present invention, the pH of the synthesis solution is determined by measuring the pH of the synthesis solution with stirring using a pH measuring device or pH test paper.

The unsaturated monomer used in the method for producing the prolate-spheroidal polymer particles A is not particularly limited as long as the above-mentioned polymer component can be synthesized from the unsaturated monomer. Examples of the preferable unsaturated monomers include a styrene monomer, a (meth)acrylate ester monomer, a carboxylic acid vinyl ester monomer, a N-vinyl compound monomer, an olefin monomer, a fluorinated olefin monomer, a conjugated diene monomer, an ionic functional group-containing monomer (hereinafter, these are collectively referred to as unsaturated monomer A1). These can be used singly or in combination of two or more types.

Examples of the styrene monomer include styrene, o-methylstyrene, m-methylstyrene, p-methylstyrene, α-methylstyrene, o-ethylstyrene, m-ethylstyrene, p-ethylstyrene, 2,4-dimethylstyrene, p-n-butylstyrene, p-tert-butylstyrene, p-n-hexylstyrene, p-n-octylstyrene, p-n-nonylstyrene, p-n-decylstyrene, p-n-dodecylstyrene, p-methoxystyrene, p-phenylstyrene, p-chlorostyrene, and 3,4-dichlorostyrene.

Examples of the (meth)acrylate ester monomer include hydrocarbon group-containing (meth)acrylic monomers such as methyl (meth)acrylate, ethyl (meth)acrylate, propyl (meth)acrylate, n-butyl (meth)acrylate, isobutyl (meth)acrylate, pentyl (meth)acrylate, hexyl (meth)acrylate, 2-ethylhexyl (meth)acrylate, octyl (meth)acrylate, nonyl (meth)acrylate, decyl (meth)acrylate, dodecyl (meth)acrylate, lauryl (meth)acrylate, stearyl (meth)acrylate, cyclohexyl (meth)acrylate, isobornyl (meth)acrylate, phenyl (meth)acrylate, toluyl (meth)acrylate, and benzyl (meth)acrylate; fluorine-containing (meth)acrylic monomers such as 2,2,2-trifluoroethyl (meth)acrylate, 3,3,3-trifluoropropyl (meth)acrylate, 2-(perfluoroethyl)ethyl (meth)acrylate, 2-perfluoroethyl-2-perfluorobutylethyl (meth)acrylate, 2-perfluoroethyl (meth)acrylate, tetrafluoropropyl (meth)acrylate, perfluoromethyl (meth)acrylate, 1,1,1,3,3,3-hexafluoropropan-2-yl (meth)acrylate, 2-perfluoromethyl-2-perfluoroethyl methyl (meth)acrylate, 2-(perfluorohexyl) ethyl (meth)acrylate, 2-(perfluorodecyl) ethyl (meth)acrylate, and 2-(perfluorohexadecyl) ethyl (meth)acrylate; alkylamino group-containing (meth)acrylic monomers such as N-propylaminoethyl (meth)acrylate, N-ethylaminopropyl (meth)acrylate, N-phenylaminoethyl (meth)acrylate, and N-cyclohexylaminoethyl (meth)acrylate; silicon-containing (meth)acrylic monomers such as γ-(methacryloyloxypropyl) trimethoxysilane and γ-(methacryloyloxypropyl) dimethoxymethylsilane; alkoxy group-containing (meth)acrylic monomers such as (poly)ethylene glycol mono (meth)acrylate, 2-methoxyethyl (meth)acrylate, and 3-methoxybutyl (meth)acrylate; (poly)alkylene glycol (meth)acrylic monomers such as (poly)propylene glycol mono (meth)acrylate; alkoxy (poly)alkylene glycol (meth)acrylic monomers such as methoxy (poly)ethylene glycol mono (meth)acrylate and methoxy (poly)propylene glycol mono (meth)acrylate; 2-chloroethyl (meth)acrylate and methyl α-chloro (meth)acrylate.

Examples of the carboxylic acid vinyl ester monomer include vinyl acetate, vinyl propionate, vinyl benzoate, vinyl butyrate, vinyl formate, vinyl valerate, and vinyl pivalate.

Examples of the N-vinyl compound monomer include N-vinylpyrrole, N-vinylcarbazole, N-vinylindole, and N-vinylpyrrolidone.

Examples of the olefin monomer include ethylene and propylene. Examples of the fluorinated olefin monomer include vinyl fluoride, vinylidene fluoride, tetrafluoroethylene, and hexafluoropropylene. Examples of the conjugated diene monomer include butadiene and isoprene.

Examples of the ionic functional group-containing monomer include a monomer having an anionic functional group (for example, sodium p-styrene sulfonate) such as a sulfonic acid group, a phosphoric acid group, and a phenolic hydroxyl group, or a cationic functional group such as an amino group, an imidazole group, a pyridine group, and an amidino group. Specific examples include sodium p-styrenesulfonate.

Among these, a styrene monomer, a (meth)acrylate ester monomer, and a carboxylic acid vinyl ester monomer are preferable. If these monomers are used, prolate-spheroidal polymer particles having the above-mentioned shape can be easily obtained at low cost.

In the method for producing the prolate-spheroidal polymer particles of the present invention, as monomers other than the unsaturated monomer A1, a multifunctional unsaturated monomer, an unsaturated monomer containing a reactive functional group such as a hydrophilic functional group and an active hydrogen group (hereinafter referred to as unsaturated monomer A2) can be used. In particular, if the crosslinking step described later is performed, the unsaturated monomer A2 needs to be used.

Examples of the reactive functional group include an epoxy group, a carboxy group, an amide group, a hydroxy group, an amino group, an alkylene oxide group, a thiol group, a carbonyl group, an ether group, a cyano group, an isocyanate group, a carbodiimide group, and an oxazoline group. Among them, an epoxy group, a carboxy group, an amide group, a hydroxy group, an amino group, a thiol group are preferable.

The unsaturated monomer A2 containing a reactive functional group include the following. In the following

description, "$C_n$" means that the number of the carbon atom is n.

(1) Multifunctional unsaturated monomer

Examples of the multifunctional unsaturated monomer include aromatic divinyl compound such as divinylbenzene, divinylbiphenyl, and divinylnaphthalene; (poly)alkylene glycol di(meth)acrylates such as (poly)ethylene glycol di(meth)acrylate, (poly)propylene glycol di(meth)acrylate, and (poly)tetramethylene glycol di(meth)acrylate; alkanediol di(meth)acrylates such as 1,6-hexanediol di(meth)acrylate, 1,8-octanediol di(meth)acrylate, 1,9-nonanediol di(meth)acrylate, 1,10-decanediol di(meth)acrylate, 1,12-dodecanediol di(meth)acrylate, 3-methyl-1,5-pentanediol di(meth)acrylate, 2,4-diethyl-1,5-pentanediol di(meth)acrylate, butylethylpropanediol di(meth)acrylate, 3-methyl-1,7-octanediol di(meth)acrylate, and 2-methyl-1,8-octanediol di(meth)acrylate; multifunctional (meth)acrylates such as glycerin di(meth)acrylate, pentaerythritol tri(meth)acrylate, pentaerythritol tetra(meth)acrylate, pentaerythritol di(meth)acrylate, pentaerythritol tetra(meth)acrylate, glycerolacryloxy di(meth)acrylate, ethoxylated cyclohexane dimethanol di(meth)acrylate, ethoxylated bisphenol A di(meth)acrylate, tricyclodecane dimethanol di(meth)acrylate, propoxylated ethoxylated bisphenol A di(meth)acrylate, 1,1,1 -trishydroxymethylethanedi(meth)acrylate, 1,1,1-trishydroxymethylethanetri(meth)acrylate, 1,1,1-trishydroxymethylpropane tri(meth)acrylate, caprolactone modified dipentaerythritol hexa(meth)acrylate, caprolactone modified hydroxypivalate ester neopentyl glycol di(meth)acrylate, polyester (meth)acrylate, and urethane (meth)acrylate; and compounds such as N,N-divinylaniline, divinyl ether, divinyl sulfide, and divinyl sulfone. These can be used singly or in combination of two or more types.

(2) Epoxy group-containing unsaturated monomer

Examples of the epoxy group-containing unsaturated monomer include epoxy group-containing (meth)acrylates such as glycidyl (meth)acrylate, (β-methyl) glycidyl (meth)acrylate, 3,4-epoxycyclohexyl (meth)acrylate, glycidyl α-ethyl acrylate, glycidyl α-n-propyl acrylate, glycidyl α-n-butyl acrylate, 3,4-epoxybutyl acrylate, 3,4-epoxybutyl methacrylate, 4,5-epoxypentyl methacrylate, 6,7-epoxyheptyl acrylate, 6,7-epoxyheptyl methacrylate, and 6,7-epoxyheptyl α-ethyl acrylate; vinyl glycidyl ethers such as o-vinylphenyl glycidyl ether, m-vinylphenyl glycidyl ether, p-vinylphenyl glycidyl ether, o-vinylbenzyl glycidyl ether, m-vinyl benzyl glycidyl ether, and p-vinyl benzyl glycidyl ether; monomers containing an ethylenic unsaturated bond and an epoxy group such as 2,3-diglycidyloxystyrene, 3,4-diglycidyloxystyrene, 2,4-diglycidyloxystyrene, 3,5-diglycidyloxystyrene, 2,6-diglycidyloxystyrene, 5-vinyl pyrogallol triglycidyl ether, 4-vinyl pyrogallol triglycidyl ether, vinylphloroglycinol triglycidyl ether, 2,3-dihydroxymethylstyrene diglycidyl ether, 3,4-dihydroxymethylstyrene diglycidyl ether, 2,4-dihydroxymethylstyrene diglycidyl ether, 3,5-dihydroxymethylstyrene diglycidyl ether, 2,6-dihydroxymethylstyrene diglycidyl ether, 2,3,4-trihydroxymethylstyrene triglycidyl ether, 1,3,5-trihydroxymethylstyrene triglycidyl ether, allyl glycidyl ether, 3,4-epoxyvinylcyclohexane, di(β-methyl) glycidyl malate, and di(β-methyl) glycidyl fumarate.

(3) Carboxy group-containing unsaturated monomer

Examples of the carboxy group-containing unsaturated monomer include unsaturated carboxylic acids such as acrylic acid, methacrylic acid, crotonic acid, cinnamic acid, itaconic acid, maleic acid, and fumaric acid; itaconic acid mono $C_1$-$C_8$ alkyl esters such as monobutyl itaconate; maleic acid mono $C_1$-$C_8$ alkyl esters such as monobutyl maleate; and vinyl group-containing aromatic carboxylic acids such as vinyl benzoate, and salts thereof.

(4) Amide group-containing unsaturated monomer

Examples of the amide group-containing unsaturated monomer include (meth)acrylamide, α-ethyl (meth)acrylamide, N-methyl (meth)acrylamide, N-butoxymethyl (meth)acrylamide, diacetone (meth)acrylamide, N,N-dimethyl (meth)acrylamide, N,N-diethyl (meth)acrylamide, N,N-dimethyl-p-styrenesulfonamide, N,N-dimethylaminoethyl (meth)acrylamide, N,N-diethylaminoethyl (meth)acrylamide,
N,N-dimethylaminopropyl (meth)acrylamide, and N,N-diethylaminopropyl (meth)acrylamide.

(5) Hydroxy group-containing unsaturated monomer

Examples of the hydroxy group-containing unsaturated monomer include hydroxy group-containing (meth)acrylic monomers such as 2-hydroxyethyl (meth)acrylate, 2-hydroxypropyl (meth)acrylate, 3-hydroxypropyl (meth)acrylate, and 4-hydroxybutyl (meth)acrylate; polyalkylene glycol (meth)acrylic monomers such as polyethylene glycol mono (meth)acrylate and polypropylene glycol mono (meth)acrylate; hydroxyalkyl vinyl ether monomers such as hydroxyethyl vinyl ether and hydroxybutyl vinyl ether; and hydroxyl group-containing allyl monomers such as allyl alcohol and 2-hydroxyethyl allyl ether.

(6) Amino group-containing unsaturated monomer

Examples of the amino group-containing unsaturated monomer include allylamine monomers such as allylamine and N-methylallylamine; amino group-containing styrene monomers such as p-aminostyrene; amino group-containing acrylic monomers such as 2-aminoethyl (meth)acrylate and 2-(dimethylamino) ethyl methacrylate; and triazine-containing monomers such as 2-vinyl-4,6-diamino-S-triazine. Among these, compounds having a primary or secondary amino group are preferable.

(7) Thiol (mercapto) group-containing unsaturated monomer

Examples of the thiol (mercapto) group-containing unsaturated monomer include mercapto group-containing (meth)acrylic monomers such as N-(2-mercaptoethyl)acrylamide, N-(2-mercapto-1-carboxyethyl)acrylamide, N-(2-mercaptoethyl)methacrylamide, N-(4-mercaptophenyl) acrylamide, N-(7-mercaptonaphthyl)acrylamide, mono 2-mercaptoethylamide maleate, 2-mercaptoethyl (meth)acrylate, and 2-mercapto-1-carboxyethyl (meth)acrylate.

(8) Carbonyl group-containing unsaturated monomer

Examples of the carbonyl group-containing unsaturated monomer include vinyl group-containing ketones such as vinyl methyl ketone, vinyl hexyl ketone, and methyl isopropenyl ketone.

(9) Ether group-containing unsaturated monomer

Examples of the ether group-containing unsaturated monomer include vinyl group-containing ether monomers such as vinyl methyl ether, vinyl ethyl ether, and vinyl isobutyl ether.

(10) Cyano group-containing unsaturated monomer

Examples of the cyano group-containing unsaturated monomer include acrylonitrile, methacrylonitrile, hexenenitrile, 4-pentenenitrile, and p-cyanostyrene.

The unsaturated monomer A2 can contain one of the reactive functional groups, or two or more of the reactive functional groups. The unsaturated monomer A2 can be used singly or in combination of two or more types.

Among the unsaturated monomer A2, aromatic divinyl compound such as divinylbenzene, divinylbiphenyl, and divinylnaphthalene; (poly)alkylene glycol di(meth)acrylates such as (poly)ethylene glycol di(meth)acrylate, (poly)propylene glycol di(meth)acrylate, and (poly)tetramethylene glycol di(meth)acrylate; alkanediol di(meth)acrylates such as 1,6-hexanediol di(meth)acrylate, 1,8-octanediol di(meth)acrylate, 1,9-nonanediol di(meth)acrylate, 1,10-decanediol di(meth)acrylate, 1,12-dodecanediol di(meth)acrylate, 3-methyl-1,5-pentanediol di(meth)acrylate, 2,4-diethyl-1,5-pentanediol di(meth)acrylate, butylethylpropanediol di(meth)acrylate, 3-methyl-1,7-octanediol di(meth)acrylate, and 2-methyl-1,8-octanediol di(meth)acrylate; hydroxyalkyl (meth)acrylate such as glycidyl (meth)acrylate, (meth)acrylic acid, (meth)acrylamide, N-methyl (meth)acrylamide, N, N-dimethyl (meth)acrylamide, N, N-diethyl (meth)acrylamide, 2-hydroxyethyl (meth)acrylate, and 3-hydroxypropyl (meth)acrylate; polyalkylene glycol (meth)acrylates such as polyethylene glycol (meth)acrylate and polypropylene glycol (meth)acrylate; p-aminostyrene, 2-aminoethyl (meth)acrylate and the like are preferable, and (poly)alkylene glycol di(meth)acrylates such as divinylbenzene, (poly)ethylene glycol di(meth)acrylate, (poly)propylene glycol di(meth)acrylate, and (poly)tetramethylene glycol di(meth)acrylate; hydroxyalkyl (meth)acrylates such as glycidyl (meth)acrylate, (meth)acrylic acid, (meth)acrylamide, 2-hydroxyethyl (meth)acrylate, and 3-hydroxypropyl (meth)acrylate are more preferable.

The unsaturated monomers A1 and A2 can be used each singly, or the unsaturated monomers A1 and A2 can be used in combination as appropriate. If the unsaturated monomers A1 and A2 are used in combination, the amount of the unsaturated monomers A1 and A2 used is preferably 99:1 to 1:99 in molar ratio (A1:A2), and from the viewpoint of taking advantage of the characteristics of the reactive functional group, it is more preferably 95:5 to 5:95, further preferably 85:15 to 15:85, and most preferably 80:20 to 20:80. If the amount of each monomer used is within the above-mentioned range, highly reactive particles for obtaining crosslinked particles and a resin layer bound on the particle surface layer described later tend to be obtained.

As a solvent for synthesis, a mixed solvent of water, a hydrophilic organic solvent, and a hydrophobic organic solvent is used. The solvent can be selected appropriately from general solvents according to the raw materials used and the like. In the present invention, a hydrophilic organic solvent means a solvent that maintains homogeneous appearance when it is mixed with water, and a hydrophobic organic solvent means a solvent that does not maintain homogeneous appearance when it is gently mixed with the same volume of pure water at a temperature of 20°C at 1 atm ($1.013 \times 10^5$ Pa), and the flowing of the mixture is stopped.

Specific examples of the solvent that can be used include water, ion exchanged water, and distilled water, and examples of the hydrophilic organic solvent include methanol, ethanol, 1-propanol, 2-propanol, ethylene glycol, propylene glycol, butylene glycol, dipropylene glycol, methyl cellosolve, ethyl cellosolve, propyl cellosolve, methyl cellosolve acetate, ethyl cellosolve acetate, methyl carbitol, ethyl carbitol, butyl carbitol, ethyl carbitol acetate, acetone, tetrahydrofuran, dimethylformamide, N-methyl-2-pyrrolidone, and acetonitrile. These can be used singly or as a mixture of two or more types.

Examples of the hydrophobic organic solvent include higher alcohols such as 1-butanol, 2-butanol, isobutanol, tert-butanol, 1-pentanol, 2-pentanol, 3-pentanol, 2-methyl-1-butanol, isopentyl alcohol, tert-pentyl alcohol, 1-hexanol, 2-methyl-1-pentanol, 4-methyl-2-pentanol, 2-ethylbutanol, 1-heptanol, 2-heptanol, 3-heptanol, 2-octanol, 2-ethyl-1-hexanol, benzyl alcohol, and cyclohexanol; ether alcohols such as butyl cellosolve; polyethers such as polypropylene glycol and polybutylene glycol; ketones such as methyl ethyl ketone, methyl isobutyl ketone, and cyclohexanone; esters such as ethyl acetate, butyl acetate, ethyl propionate, and butyl carbitol acetate; aliphatic or aromatic hydrocarbons such as pentane, 2-methylbutane, n-hexane, cyclohexane, 2-methylpentane, 2,2-dimethylbutane, 2,3-dimethylbutane, heptane, n-octane, isooctane, 2,2,3-trimethylpentane, decane, nonane, cyclopentane, methylcyclopentane, methylcyclohexane, ethylcyclohexane, p-menthane, dicyclohexyl, benzene, toluene, xylene, ethylbenzene, liquid paraffin, mineral oil, and oil for a heat medium; siloxane compounds such as polydimethylsiloxane, polymethylphenylsiloxane, polydiphenylsiloxane, and silicone oil; halogenated hydrocarbons such as carbon tetrachloride, trichloroethylene, chlorobenzene, and tetrabromoethane. These hydrophobic organic solvents can contain modified compounds substituted with carbon, nitrogen, oxygen, hydrogen, halogen or the like, and modified polymer compounds such as copolymers as long as the effects of the present invention are not impaired. These can be used singly or as a mixture of two or more types.

Among the hydrophobic organic solvents, it is preferable to use a hydrophobic organic solvent having 8 or more carbon atoms that does not react with the raw material unsaturated monomer under the polymerization conditions. If such a hydrophobic organic solvent coexists in the reaction system, the dispersibility of the produced prolate-spheroidal polymer particles can be improved, and the particle size can be controlled more uniformly.

The organic compound having 8 or more carbon atoms is not particularly limited as long as it is a liquid at least at a temperature at which polymerization is carried out and does not adversely affect the formation of polymer particles. However, the organic compound is preferably one having a melting point of 80°C or less, preferably 60°C or less, more preferably 40°C or less, and still more preferably 30°C or less. Examples of the organic compound include aliphatic or aromatic hydrocarbons such as n-octane, isooctane, 2,2,3-trimethylpentane, decane, nonane, cyclopentane, methylcyclopentane, methylcyclohexane, ethylcyclohexane, p-menthane, dicyclohexyl, benzene, toluene, xylene, ethylbenzene, liquid paraffin, mineral oil, and oil for a heat medium; siloxane compounds such as polydimethylsiloxane, polymethylphenylsiloxane, polydiphenylsiloxane, and silicone oil; polyethers such as polypropylene glycol and polybutylene glycol. The number of carbon atoms can be 8 or more. However, in consideration of the dispersion stability of the particles obtained, the number of carbon atoms is preferably 10 or more, more preferably 12 or more, and optimally 15 or more.

Further, the molecular weight of the hydrophobic organic solvent is preferably 200 or more, more preferably 300 or more, still more preferably 500 or more, and optimally 1,000 or more. Such a hydrophobic organic solvent having high molecular weight acts also like a dispersant, and inhibits the sticking and aggregation of particles, thereby prolate-spheroidal polymer particles that are monodisperse and have controlled particle size can be stably obtained.

As the hydrophobic organic solvent having a molecular weight of 200 or more, a polymer compound having a repeating unit is preferable. Specific examples include siloxane compounds such as polydimethylsiloxane, polymethylphenylsiloxane, polydiphenylsiloxane, and silicone oil; polyethers such as polypropylene glycol and polybutylene glycol; and aliphatic or aromatic hydrocarbons such as liquid paraffin and oil for a heat medium. In particular, it is further preferable that the polymer compound is a polymer compound that is water-soluble in its low molecular state and exhibits hydrophobicity as it polymerizes, or a hydrophobic organic solvent obtained by polymerizing a monomer having a polar group within the molecule. If such a polar group is contained within the molecule, a polymer stabilizer described later is easily dispersed uniformly in the solvent, which contributes to the further particle stability. Examples of the polar group include a hydroxy group, an ether group, and a carbonyl group.

Specific examples of the preferable hydrophobic organic solvent include polyethers such as polypropylene glycol and polybutylene glycol; and siloxane compounds such as polydimethylsiloxane, polymethylphenylsiloxane, polydiphenylsiloxane, and silicone oil.

The mixing ratio of water, the hydrophilic organic solvent, and the hydrophobic organic solvent is preferably 99:0.5:0.5 to 25:55:20, more preferably 98:1:1 to 35:50:15, and further preferably 97:2:1 to 45:45:10 in weight ratio.

If such a mixed solvent is used, a fuzzy state in which the emulsified layer (a lower layer, a water-rich layer), the dissolved layer (an intermediate layer, a hydrophilic solvent-rich layer), and the separated layer (an upper layer, a hydrophobic solvent-rich layer) coexist appears when the mixed solvent is left to stand, and it is presumed that even in the polymerization reaction, the polymerization reaction proceeds in a state in which the unsaturated monomer is dissolved in each layer in the fuzzy state. It is presumed that if the polymerization reaction proceeds in a solvent system that forms this fuzzy state, the dissolution balance of the solvent breaks down due to heat on the trigger of the polymerization by an initiator, prolate-spheroidal polymer particles are obtained in a state where the dispersion state is stabilized with the change in tension at the particle precipitation interface, at the same time, the particle growth reaction is stably promoted by setting the pH of the synthesis solution to a predetermined value, and thus the polymer particles of the object can be obtained efficiently and more stably.

Examples of the polymer stabilizer include various hydrophobic or hydrophilic stabilizers such as polystyrene derivatives such as polyethylene glycol, polyhydroxystyrene, polystyrene sulfonate, hydroxystyrene-(meth)acrylate ester copolymer, styrene-(meth)acrylate ester copolymer, and styrene-hydroxystyrene-(meth)acrylate ester copolymer; poly(meth)acrylic acid derivatives such as poly(meth)acrylic acid, poly(meth)acrylamide, polyacrylonitrile, polyethyl(meth)acrylate, and polybuty(meth)acrylate; polyethers and derivatives thereof such as polymethyl vinyl ether, polyethyl vinyl ether, polybutyl vinyl ether, polyisobutyl vinyl ether, polyhexyl vinyl ether; cellulose derivatives such as cellulose, methyl cellulose, cellulose acetate, cellulose nitrate, hydroxymethyl cellulose, hydroxyethyl cellulose, hydroxypropyl cellulose, and carboxymethyl cellulose; polyvinyl acetate derivatives such as polyvinyl alcohol, polyvinyl butyral, polyvinyl formal, and polyvinyl acetate; nitrogen-containing polymer derivatives such as polyvinylpyridine, polyvinylpyrrolidone, polyethyleneimine, and poly-2-methyl-2-oxazoline; and polyhalogenated vinyl derivatives such as polyvinyl chloride and polyvinylidene chloride. These can be used singly or in combination of two or more types.

The polymer stabilizer is preferably compounded in an appropriate amount of 0.01 to 50% by weight with respect to the raw material unsaturated monomer.

As the polymerization initiator, various known polymerization initiators can be used, and examples of the water-soluble polymerization initiator include water-soluble or ionic polymerization initiators such as persulfates such as ammonium persulfate, sodium persulfate, and potassium persulfate; and azo initiators such as 2,2'-azobis[2-methyl-N-(2-hydroxyethyl)propionamide], 2,2'-azobis(2-amidinopropane)dihydrochloride, 2,2'-azobis(2-methyl-N-phenylpropionamidine)dihydrochloride, 2,2'-azobis[N-(4-chlorophenyl)-2-methylpropionamidine] dihydrochloride, 2,2'-azobis[N-(4-hydroxyphenyl)-2-methylpropionamidine] dihydrochloride, 2,2'-azobis[N-(4-amino-phenyl)-2-methylpropionamidine] tetrahydrochloride, 2,2'-azobis[2-methyl-N(phenylmethyl)propionamidine] dihydrochloride, 2,2'-azobis[2-methyl-N-2-propenylpropionamidine] dihydrochloride, 2,2'-azobis[N-(2-hydroxyethyl)-2-methylpropionamidine]dihydrochloride, 2,2'-azobis[2-(5-methyl-2-imidazolin-2-yl)propane]dihydrochloride, 2,2'-azobis[2-(2-imidazolin-2-yl)propane]dihydrochloride, 2,2'-azobis[2-(4,5,6,7-tetrahydro-1H-1,3-diazepin-2-yl)propane]dihydrochloride, 2,2'-azobis[2-(3,4,5,6-tetrahydropyrimidin-2-yl)propane]dihydrochloride, 2,2'-azobis[2-(5-hydroxy-3,4,5,6-tetrahydropyrimidin-2-yl)propane]dihydrochloride, 2,2'-azobis{2-[1-(2-hydroxyethyl)-2-imidazolin-2-yl]propane}dihydrochloride, 2,2'-azobis-2-cyanopropane-1-sulfonic acid disodium salt, and 4,4'-azobis(4-cyanopentanoic acid)sodium salt.

Meanwhile, examples of an oil initiator include oil-soluble polymerization initiators such as peroxides such as benzoyl peroxide, cumene hydroperoxide, and tert-butyl hydroperoxide; azo compounds such as azobisisobutyronitrile, azobismethylbutyronitrile, azobisisovaleronitrile, 2,2'-azobis(dimethyl isobutyrate), 2,2'-azobis(N-butyl-2-methylpropionamide), 4,4'-azobis(4-cyanopentanoic acid), 2,2'-azobis(2-amidinopropane)dihydrochloride, and 2,2'-azobis(N,N'-dimethyleneisobutylamidine)dihydrochloride.

These polymerization initiators can be used singly or as a mixture of two or more types. It is preferable that the compounding amount of the radical polymerization initiator be usually 0.01 to 50 parts by weight per 100 parts by weight of the raw material unsaturated monomer.

Among these, if an initiator such as a persulfate is used, the initiator not only functions as a radical initiator but also produces an acid by hydrolysis and the like, and the pH of the synthesis solution can be adjusted without separately adding a pH adjuster. The addition amount is preferably 5 to 30% by weight.

Adjusting the components and compositions of water, the hydrophilic organic solvent, and the hydrophobic organic solvent as appropriate allows the prolate-spheroidal polymer particles to have characteristics such as fine irregularities on the surface, porosity, and large specific surface area. Thus, the surface and the inside of the particles can be appropriately reformed.

In the present invention, by adjusting the solvent composition as described above, the particle size and aspect ratio of the prolate-spheroidal polymer particles, the size of fine irregularities on the surface, and the porosity can be controlled more stably, and thus, various properties such as water absorption and oil absorption can be controlled with balance according to the use.

In the production of the prolate-spheroidal polymer particles A, an emulsifier (a surfactant) or the like can be compounded in an appropriate amount of 0.01 to 50% by weight with respect to the raw material unsaturated monomer as needed.

Examples of the emulsifier (the surfactant) include anionic emulsifiers such as alkyl sulfate salts such as sodium dodecyl sulfate, alkyl benzene sulfonates such as sodium dodecyl benzene sulfonate, alkyl naphthalene sulfonates, fatty acid salts, alkyl phosphates, and alkyl sulfosuccinates; cationic emulsifiers such as alkylamine salts, quaternary ammonium salts, alkylbetaines, and amine oxides; and nonionic emulsifiers such as polyoxyethylene alkyl ether, polyoxyethylene alkyl allyl ether, polyoxyethylene alkyl phenyl ether, sorbitan fatty acid ester, glycerin fatty acid ester, sucrose fatty acid ester, and polyoxyethylene fatty acid ester. These can be used singly or in combination of two or more types.

The long diameter and the short diameter of the prolate-spheroidal polymer particles can be controlled by addition of an emulsifier. Further, one or more emulsifiers that are solid at normal temperature is preferably included.

The content of the raw material unsaturated monomer in the synthesis solution is preferably 1 to 80% by weight, more preferably 5 to 50% by weight, and further preferably 10 to 40% by weight in the total synthesis solution. If the content of the raw material unsaturated monomer is more than 80% by weight, aggregates may increase, and obtaining polymer particles having the above-mentioned physical properties in a monodispersed state in a high yield may be difficult. Meanwhile, if the content is less than 1% by weight, it takes a long time to complete the reaction, and it is not practical from an industrial viewpoint.

The reaction temperature at the time of polymerization varies depending on the type of the solvent used, and cannot be generally defined. However, it is usually about 10 to 200°C, preferably 30 to 130°C, and more preferably 40 to 90°C.

The reaction time is not particularly limited as long as it is a time required for the target reaction to be substantially completed. The reaction time depends largely on the kind of the unsaturated monomer and its compounding amount, the viscosity and concentration of the solution, the particle size of the particles of the object and the like. For example, at 40 to 90°C, the reaction time is about 1 to 72 hours, and preferably about 2 to 24 hours.

In the polymerization reaction, a catalyst (a reaction accelerator) can be compounded according to the use and the like of the particles obtained. The compounding amount can be an appropriate amount that does not adversely affect the particle physical properties, for example, 0.01 to 20% by weight with respect to the total weight of the polymerization components.

The catalyst is not particularly limited as long as it is a positive catalyst, and can be appropriately selected from known catalysts and used. Specific examples include catalysts such as tertiary amines such as benzyldimethylamine, triethylamine, tributylamine, pyridine, and triphenylamine; quaternary ammonium compounds such as triethylbenzyl ammonium chloride and tetramethyl ammonium chloride; phosphines such as triphenyl phosphine and tricyclo phosphine; phosphonium compounds such as benzyltrimethylphosphonium chloride; imidazole compounds such as 2-methylimidazole and 2-methyl-4-ethylimidazole; alkali metal hydroxides such as potassium hydroxide, sodium hydroxide, and lithium hydroxide; alkali metal carbonates such as sodium carbonate and lithium carbonate; alkali metal salts of organic acids; and Lewis acid halides such as boron trichloride, boron trifluoride, tin tetrachloride, and titanium tetrachloride, or complex salts thereof. These can be used singly or in combination of two or more types.

In the polymerization reaction, in order to adjust the size, shape, quality and the like of the prolate-spheroidal polymer particles obtained, a compound which can be dissolved in water or other polar solvents, ionizes into positive and negative ions, and provides electrical conductivity to the solution can be added.

Specific examples of the compound include salts, inorganic acids, inorganic bases, organic acids, organic bases, and ionic liquids. The compounding amount can be an appropriate amount that does not adversely affect the particle physical properties, for example, 0.01 to 80% by weight with respect to the total weight of the polymerization components.

By changing the type and amount ratio of the unsaturated monomer, the polymer stabilizer, and the solvent, and the amount ratio of the dispersing agent and the emulsifier to be used as needed, the prolate-spheroidal polymer particles having different particle size, aspect ratio, shape and the like can be produced more stably in a monodispersed state.

After the solution polymerization step, the obtained prolate-spheroidal polymer particles and a crosslinker having a reactive group which reacts with the reactive functional group can be crosslinked by reacting the functional group contained in the prolate-spheroidal polymer particles with the reactive group contained in the crosslinker in the presence of a solvent in which the prolate-spheroidal polymer particles are soluble but the crosslinker is insoluble. At this time, the obtained prolate-spheroidal polymer particles are the particles that have been obtained using the unsaturated monomer A2. Crosslinking can be performed, for example, by the method described in Patent Document 5.

[Polymer particles B]

The polymer particles (A) can include, in addition to the prolate-spheroidal polymer particles A, polymer particles B having a shape different from a shape of the prolate-spheroidal polymer particles A. Examples of the different shape described herein include a flat ellipsoidal shape, a sphere, an approximate sphere, a crushed shape, a polyhedron, an irregular shape, an aggregated shape and the like.

The polymer particles B is not particularly limited as long as it has a shape different from a shape of the prolate-spheroidal polymer particles A. In particular, the polymer particles B are flat ellipsoidal polymer particles that have a front view, a plan view and a side view of projections according to third angle projection that are all ellipses and that satisfy (6) to (8) below:

(6) an average ($L^2_{AV}$) of a long diameter ($L^2$) of a flat part of $0.13 \leq L^2_{AV} \leq 500$ μm;
(7) an average ($D^2_{AV}$) of a short diameter ($D^2$) of a flat part of $0.1 \leq D^2_{AV} \leq 250$ μm; and
(8) an average ($P^2_{AV}$) of an aspect ratio ($L^2/D^2$) calculated from the long diameter ($L^2$) and the short diameter ($D^2$) of $1.3 < P^2_{AV} \leq 50$.

In the present invention, the "flat ellipsoidal" includes not only a shape of an elliptical sphere (an ellipsoid) that is flattened in mathematical meaning, but also an approximate ellipsoidal shape having a flat part, for example, an approximate flat ellipsoidal shapes such as those having a flat part that is a rectangle with rounded corners and those having a flat part that is oval.

The flat ellipsoidal polymer particles preferably have $L^2_{AV}$ of $1 \leq L^2_{AV} \leq 300$ μm, more preferably $5 \leq L^2_{AV} \leq 150$ μm, and still more preferably $8 \leq L^2_{AV} \leq 100$ μm in consideration of the fine finish of the makeup. The flat ellipsoidal polymer particles preferably have $D^2_{AV}$ of $0.1 \leq D^2_{AV} \leq 150$ μm, more preferably $0.5 \leq D^2_{AV} \leq 100$ μm, and still more preferably $0.8 \leq D^2_{AV} \leq 80$ μm. The flat ellipsoidal polymer particles preferably have $P^2_{AV}$ of $1.4 \leq P^2_{AV} \leq 40$, more preferably $1.6 \leq P^2_{AV} \leq 35$, and still more preferably $1.8 \leq P^2_{AV} \leq 30$.

The flat ellipsoidal polymer particles preferably have a shape that further satisfies at least one of (9) and (10) below because the larger the contact area between the particles and the solid surface, the greater the adhesion, and the smaller the resistance to the fluid, the less likely the detachment or peeling off of particles:

(9) an average ($P^3_{AV}$) of an aspect ratio ($D^2/T^2$) calculated from the short diameter ($D^2$) and the thickness ($T^2$) of the side of $1.2 < P^3_{AV} \leq 100$.
(10) an average ($P^4_{AV}$) of an aspect ratio ($L^2/T^2$) calculated from the long diameter ($L^2$) and the thickness ($T^2$) of $1.56 < P^4_{AV} \leq 150$.

$P^3_{AV}$ is preferably $1.3 \leq P^3_{AV} \leq 50$, more preferably $1.5 \leq P^3_{AV} \leq 30$, and still more preferably $2 \leq P^3_{AV} \leq 20$. $P^4_{AV}$ is preferably $1.7 \leq P^4_{AV} \leq 100$, more preferably $2 \leq P^4_{AV} \leq 50$, and still more preferably $3 \leq P^4_{AV} \leq 30$.

The flat ellipsoidal polymer particles preferably have at least any of characteristics of fine irregularities on the particle surface, porosity, and relatively large specific surface area. In particular, flat ellipsoidal polymer particles that are porous or flat ellipsoidal polymer particles in which fine particles that satisfy (11) below are attached or contained at least on the surface or in the surface layer are preferable, and flat ellipsoidal polymer particles having irregularities formed by the fine particles on the surface are more preferable.
(11) a particle size ($SP^2$) of the fine particles which are attached or contained on the surface or in the surface layer is $1/1000 \times D^2_{AV} \leq SP^2 \leq 1/2 \times D^2_{AV}$.

$SP^2$ is more preferably $1/100 \times D^2_{AV} \leq SP^2 \leq 1/2 \times D^2_{AV}$, and still more preferably $1/20 \times D^2_{AV} \leq SP^2 \leq 1/2 \times D^2_{AV}$. The fine particles are preferably composed of the component same as the component of the flat ellipsoidal polymer particles, and are preferably attached or contained on the surface or in the surface layer of the flat ellipsoidal polymer particles.

The flat ellipsoidal polymer particles preferably have a volume mean particle size ($MV^2$) of 0.1 to 500 μm, more preferably 0.5 to 100 μm, and further preferably 1 to 50 μm.

The flat ellipsoidal polymer particles preferably have a bulk density of 0.01 to 0.7 g/mL, more preferably 0.05 to 0.65 g/mL, and still more preferably 0.1 to 0.6 g/mL.

The flat ellipsoidal polymer particles preferably have affinity to at least one of water and oil, and more preferably have affinity to both of them. Specifically, it is preferable that the water absorption be 50 mL or more per 100 g of particles, and/or the oil absorption be preferably 50 mL or more per 100 g of particles, and it is more preferable that the water absorption be 60 mL or more per 100 g of particles and the oil absorption be 60 mL or more per 100 g of particles. In particular, it is more preferable that both the water absorption and the oil absorption be 80 mL or more per 100 g, and it is optimal that both the water absorption and the oil absorption be 100 mL or more per 100 g.

Further, the flat ellipsoidal polymer particles are preferably crosslinked polymer particles from the viewpoint of improving heat resistance and chemical resistance. The method of crosslinking is not particularly limited, and examples of the method include the method in which polymerization reaction is performed using a multifunctional unsaturated monomer that functions as a crosslinker. By crosslinking the polymer particles, the polymer particles having excellent heat resistance (heat resistance of 100°C or more) that maintain the initial shape after heating 0.5 g of the polymer particles at 100°C for 2 hours can be obtained.

The polymer component of the flat ellipsoidal polymer particles is not particularly limited as long as its shape is maintained. However, in consideration of the safety of the cosmetic, components generally used in cosmetic uses are preferable, such as a styrene polymer, a (meth)acrylic polymer, a polyolefin polymer, a carboxylic acid vinyl ester polymer, a poly-N-vinyl compound polymer, a polyamide polymer, a polyester polymer, a silicone polymer, and a polyurethane polymer. These polymers can be homopolymers or copolymers. Specific examples of these polymers include the same as those above-mentioned.

The flat ellipsoidal polymer particles are preferably (co)polymers with at least one monomer selected from, in particular, a styrene monomer, (meth)acrylic acid, a (meth)acrylate ester monomer, a carboxylic acid vinyl ester monomer, a N-vinyl compound monomer, an olefin monomer, a fluorinated olefin monomer, a conjugated diene monomer, and a multifunctional unsaturated monomer, and are more preferably (co)polymers that contain as an essential unit a repeating unit obtained from at least one selected from a styrene monomer, (meth)acrylic acid, and a (meth)acrylate ester. If a multifunctional unsaturated monomer is used, the polymer particles obtained can be crosslinked. Specific examples of the monomer include the same as those above-mentioned.

The flat ellipsoidal polymer particles can be used singly or in combination of two or more types that satisfy the conditions (6) to (8).

The flat ellipsoidal polymer particles can be produced, for example, according to the method described in WO 2016/181877.

If polymer particles other than the above-mentioned flat ellipsoidal polymer particles are used as polymer particles B, the polymer particles preferably satisfy the volume mean particle size ($MV^3$) of $1/5 \times D^1_{AV} \leq MV^3 \leq L^1_{AV}$, more preferably satisfy the volume mean particle size of $1/3 \times D^1_{AV} \leq MV^3 \leq 0.8 \times L^1_{AV}$, still more preferably satisfy the volume mean particle size of $1/2 \times D^1_{AV} \leq MV^3 \leq 0.6 \times L^1_{AV}$, and further preferably $D^1_{AV} \leq MV^3 \leq 1/2 \times L^1AV$.

The polymer particles other than the flat ellipsoidal polymer particles are preferably spherical or approximately spherical. The polymer component that constitutes such polymer particles are preferably composed of a component same as that of the prolate-spheroidal polymer particles A, or at least include a component same as that of the prolate-spheroidal polymer particles A from the viewpoint of efficient production. The production method is not particularly limited as long as the above-mentioned shape can be obtained, and for example, the polymer particles can be produced by grinding, solution polymerization or the like.

If two or more types of prolate-spheroidal polymer particles A are used in combination as the polymer particles (A), or if one or more types of prolate-spheroidal polymer particles A and polymer particles B are used in combination, they are preferably combined so that the combination of the polymer particles has a volume mean particle size (MV) in the particle size distribution of 1 to 25 μm and satisfies dispersion range (SD) of 1.5 to 20. SD is more preferably 2 to 20, further preferably 2.5 to 15, and most preferably 3 to 12. If MV and SD are in the above-mentioned range, the synergistic effect of each type of shapes for the properties of each type of the polymer particles can be obtained.

If two or more types of prolate-spheroidal polymer particles A are used in combination as the polymer particles (A), and/or if one or more types of prolate-spheroidal polymer particles A and polymer particles B are used in combination, they are preferably combined so that the combination of polymer particles has a difference (MV - MN) between a volume mean particle size (MV) and a number mean particle size (MN) of 1.5 μm or more. MV - MN is more preferably 2 μm or more, further preferably 2.5 μm or more, and most preferably 3 μm or more. If MV - MN is in the above-mentioned range, the synergistic effect for properties resulting from the shape of each type of the polymer particles can be obtained.

If two or more types of polymer particles are used in combination as the polymer particles (A), they are combined so that the combination of the polymer particles satisfies at least one of (12), (13), and (14) below:

(12) a difference between a maximum and a minimum of the average ($P^2_{AV}$ in the case of the flat ellipsoidal polymer particles) of an aspect ratio of two or more;
(13) a difference between a maximum and a minimum of a volume mean particle size of 3 μm or more; and
(14) appearance of two or more peaks in particle size distribution measurement.

From the viewpoint of obtaining a higher synergistic effect, the polymer particles are more preferably combined so that the combination satisfies two or more of (12), (13), and (14) above-mentioned, and most preferably combined so that the combination satisfies all of (12), (13), and (14). For the condition (12), from the viewpoint of obtaining a higher synergistic effect, the combination preferably satisfies the difference between the maximum and the minimum of the average of the aspect ratio of 3 or more, and further preferably satisfies the difference between the maximum and the minimum of the average of the aspect ratio of 4 or more. For the condition (13), from the viewpoint of obtaining a higher synergistic effect, the combination preferably satisfies the difference between the maximum and the minimum of the volume mean particle size of 4 μm or more, and further preferably the difference between the maximum and the minimum of the volume mean particle size of 5 μm or more.

As described above, by combining two or more types of polymer particles, a higher synergistic effect for the properties of each type of the polymer particles can be obtained.

If the prolate-spheroidal polymer particles A and the polymer particles B are used as the polymer particles (A), the compounding ratio of the prolate-spheroidal polymer particles A to the polymer particles B is preferably 99 : 1 to 10 : 90 in weight ratio.

The skin cosmetic of the present invention has the content of the polymer particles (A) of 0.1 to 40% by weight. The lower limit of the content is preferably 0.5% by weight, more preferably 1% by weight, and further preferably 2% by weight. Meanwhile, the upper limit of the content is preferably 30% by weight, more preferably 20% by weight, and further preferably 15% by weight. The content of the polymer particles can be appropriately adjusted within the above-mentioned range according to the use and purpose such as light scattering properties such as a gradation effect, UV scattering properties, fluidity, formability, improvement of adhesiveness, and finish feeling.

[Other components]

The skin cosmetic of the present invention includes, in addition to component (A), inorganic particles (B) and an oily component (C), and can further include components commonly used in cosmetics as appropriate. Hereinafter, components other than component (A) are collectively called other components. The skin cosmetic of the present invention include 60 to 99.9% by weight of other components.

[Inorganic particles (B)]

The skin cosmetic of the present invention includes inorganic particles (B) as other components from the viewpoint of appropriately adjusting adhesion to the skin, extension, gloss, coloring, hiding properties and the like. Examples of the inorganic particles include inorganic particles having various sizes and shapes such as mica, talc, kaolin, sericite, montmorillonite, kaolinite, mica, muscovite, phlogopite, synthetic mica, lepidolite, biotite, permiculite, magnesium carbonate, calcium carbonate, aluminum silicate, barium silicate, calcium silicate, magnesium silicate, strontium silicate, metal tungstate, magnesium, zeolite, barium sulfate, calcined calcium sulfate, calcium phosphate, fluoroapatite, hydroxyapatite, ceramic powder, bentonite, smectite, clay, mud, metal soap (for example, zinc myristate, calcium palmitate, and aluminum stearate), red iron oxide, yellow iron oxide, black iron oxide, ultramarine blue, prussian blue, carbon black, titanium oxide, fine particles and ultrafine particles titanium oxide, zinc oxide, fine particles and ultra fine particles zinc oxide, alumina, silica, fumed silica (ultra fine particle anhydrous silica), mica titanium, fish scale guanine, boron nitride, photochromic pigment, synthetic fluorine phlogopite, gold, aluminum, and fine particle composite powder, and the inorganic particles obtained by treating the above-mentioneed inorganic particles with various surface treatment agents such as silicones such as hydrogen silicones or cyclic hydrogen silicones, other silane compounds, and titanium coupling agents to hydrophobize or hydrophilize them.

The shape of the inorganic particles is not particularly limited, and examples include a sphere, an approximate sphere, a plate-like shape, a scaly shape, a crushed shape, a bar, a polyhedron, an irregular shape, and an aggregated shape. Among these, the plate-like shape or the scaly shape is preferable from the viewpoint of improving adhesion.

The inorganic particles preferably have a volume mean particle size of 0.01 to 300 μm, and more preferably have a volume mean particle size of 0.1 to 200 μm. If the volume mean particle size is in the above-mentioned range, smooth extension and spread at the time of application can be obtained, and soft focus properties can be improved by steric hindrance of each type of powder occurred on the skin.

The content of the inorganic particles is preferably 1 to 99% by weight, and more preferably 5 to 95% by weight in other components. If the content of the inorganic particles is in the above-mentioned range, a cosmetic effect as a cosmetic can be sufficiently obtained. The inorganic particles can be used singly or in combination of two or more types.

[(C) Oily component]

The skin cosmetic of the present invention includes an oily component (C) as other components. Examples of the oily component include higher alcohols, higher fatty acids, oils and fats, waxes, hydrocarbons, silicones, and fatty acid esters.

Specifically, preferable examples of the oily component include higher alcohols such as cetanol, myristyl alcohol, oleyl alcohol, lauryl alcohol, cetostearyl alcohol, stearyl alcohol, arakyl alcohol, behenyl alcohol, jojoba alcohol, chimyl alcohol, serakyl alcohol, batyl alcohol, hexyl decanol, isostearyl alcohol, 2-octyl dodecanol, and dimer diol; aralkyl alcohols such as benzyl alcohol and derivatives thereof; higher fatty acids such as lauric acid, myristic acid, palmitic acid, stearic acid, isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteisohenicoic acid, long chain branched fatty acids, dimer acid, and hydrogenated dimer acid, and metal soaps thereof such as aluminum salts, calcium salts, magnesium salts, zinc salts, potassium salts thereof, and nitrogen-containing derivatives such as amides; hydrocarbons such as liquid paraffin (mineral oil), heavy (liquid) isoparaffin, light (fluid) isoparaffin, α-olefin oligomer, polyisobutene (polyisobutylene), hydrogenated polyisobutene, polybutene, squalane, olive-derived squalane, squalene, vaseline, and solid paraffin; waxes such as candelilla wax, carnauba wax, rice wax, Japan wax, bees wax, montan wax, ozokerite, ceresin, paraffin wax, microcrystalline wax, petrolatum, Fischer Tropsch wax, polyethylene wax, and ethylene/propylene copolymer; vegetable fats and oils such as coconut oil, palm oil, palm kernel oil, safflower oil, olive oil, castor oil, avocado oil, sesame oil, tea oil, evening primrose oil, camellia oil, wheat germ oil, macadamia oil, hazelnut oil, candlenut oil, rosehip oil, meadowfoam oil, persic oil, tea tree oil, peppermint oil, corn oil, rapeseed oil, sunflower oil, wheat germ oil, linseed oil, cottonseed oil, soybean oil, peanut oil, rice bran oil, cocoa butter, shea butter, hydrogenated coconut oil, hydrogenated castor oil, jojoba oil, and hydrogenated jojoba oil; animal fats and oils such as beef tallow, milk fat, horse fat, egg yolk oil, mink oil, and turtle oil; animal waxes such as spermaceti wax, lanolin, and orange roughy oil; lanolins such as liquid lanolin, reduced lanolin, adsorptive purification lanolin, lanolin acetate, liquid lanolin acetate, hydroxy lanolin, polyoxyethylene lanolin, lanolin fatty acid, hard lanolin fatty acid, lanolin alcohol, lanolin acetate alcohol, and acetate (cetyl lanolyl) ester; phospholipids such as lecithin, phosphatidyl choline, phosphatidyl ethanolamine, phosphatidyl serine, phosphatidyl glycerol, phosphatidylinositol, sphingophospholipids such as sphingomyelin, phosphatidic acid, and lysolecithin; phospholipid derivatives such as hydrogenated soybean phospholipids, partially hydrogenated soybean phospholipids, hydrogenated egg yolk phospholipids, and partially hydrogenated egg yolk phospholipids; sterols such as cholesterol, dihydrocholesterol, lanosterol, dihydrolanosterol, phytosterols, and cholic acid; sapogenins; saponins; sterol esters such as cholesteryl acetate, cholesteryl nonanoate, cholesteryl stearate, cholesteryl isostearate, cholesteryl oleate, N-lauroyl-L-glutamic acid di(cholesteryl/behenyl/octyldodecyl), N-lauroyl-L-glutamic acid di(cholesteryl/octyldodecyl), N-lauroyl-L-glutamic acid di(phytosteryl/behenyl/octyldodecyl), N-lauroyl-L-glutamic acid di(phytosteryl/octyldodecyl), acyl sarcosine alkyl esters such as N-lauroyl sarcosine isopropyl, cholesteryl 12-hydroxystearate, macadamia nut oil fatty acid cholesteryl, macadamia nut oil fatty acid phytosteryl, phytosteryl isostearate, soft lanolin fatty acid cholesteryl, hard lanolin fatty acid cholesteryl, long chain branched fatty acid cholesteryl, and long chain $\alpha$-hydroxy fatty acid cholesteryl; lipid complexes such as phospholipid/cholesterol complex and phospholipid/phytosterol complex; monoalcohol carboxylic acid esters such as octyl dodecyl myristate, hexyl decyl myristate, octyl dodecyl isostearate, cetyl palmitate, octyldodecyl palmitate, cetyl octanoate, hexyldecyl octanoate, isodecyl isononanoate, isononyl isononanoate, octyl isononanonate, isotridecyl isononanoate, isodecyl neopentanoate, isotridecyl neopentanoate, isostearyl neopentanoate, octyldodecyl neodecanoate, oleyl oleate, octyldodecyl oleate, octodecyl dodecyl ricinoleate, octyl dodecyl lanolin fatty acid, hexyl decyl dimethyl octanoate, octyl dodecyl erucate, hydrogenated castor oil isostearate, ethyl oleate, avocado oil fatty acid ethyl, isopropyl myristate, isopropyl palmitate, octyl palmitate, isopropyl isostearate, 2-ethylhexyl hydroxystearate, isopropyl lanolin fatty acid, diethyl sebacate, diisopropyl sebacate, dioctyl sebacate, diisopropyl adipate, dibutyl octyl sebacate, diisobutyl adipate, dioctyl succinate, and triethyl citrate; oxyacid esters such as cetyl lactate, diisostearyl malate, and hydrogenated castor oil monoisostearate; polyhydric alcohol fatty acid esters such as glyceryl trioctanoate, glyceryl trioleate, glyceryl triisostearate, glyceryl diisostearate, glyceryl tri(tricaprylate/tricaprate), glyceryl tri(tricaprylate/tricaprate/myristate/stearate), hydrogenated rosin triglyceride (hydrogenated ester gum), rosin triglyceride (ester gum), glyceryl beicosate eicosane diacid, trimethylolpropane trioctanoate, trimethylolpropane triisostearate, neopentyl glycol dioctanoate, neopentyl glycol dicaprate, 2-butyl 2-ethyl 1,3-propanediol dioctoate, propylene glycol dioleate, pentaerythrityl tetraoctanoate, hydrogenated rosin pentaerythrityl, ditrimethylolpropane triethylhexanoate, ditrimethylolpropane (isostearate/sebacate), pentaerythrityl triethylhexanoate, dipentaerythrityl (hydroxystearate/stearate/resinate), diglyceryl diisostearate, sucrose tetraisostearate, polyglyceryl tetraisostearate, polyglyceryl-10 nonaisostearate, polyglyceryl-8 deca(erucate/isostearate/ricinoleate), (hexyl decanoic acid/sebacic acid) diglyceryl oligoester, and glycol distearate (ethylene glycol distearate); derivatives of dimer acids or dimer diols such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosinate, dimer dilinoleic acid hydrogenated castor oil, and hydroxyalkyl dimer dilinoleyl ether; fatty acid alkanolamides such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (paltamide MEA), palmitic acid diethanolamide (paltamide DEA), and coconut oil fatty acid methyl ethanolamide (cocamide methyl MEA); various silicones containing cyclic siloxanes such as methicone (monomethylpolysiloxane), dimethicone (dimethylpolysiloxane), highly polymerized dimethicone (highly polymerized dimethylpolysiloxane), methylphenylpolysiloxane, cyclomethicone (octamethylcyclotetrasiloxane), decamethylcyclopentasiloxane, tetrahydrotetramethylcyclotetrasiloxane, methyl cyclopolysiloxane, cyclopentasiloxane, and dodecamethylcyclohexasiloxane; and fluorine oils such as perfluorodecane, perfluorooctane, and perfluoropolyether.

In particular, cosmetics to be applied directly to the skin, such as foundations, and cosmetics that are desired to have adhesiveness, long lasting performance, and stability preferably have a content of the oily component of 0.1% by weight or more, more preferably 0.5% by weight or more, still more preferably 1% by weight or more, and most preferably 2% by weight or more in other components from the viewpoint of further improving the properties of the prolate-spheroidal polymer particles A, and from the viewpoint of the moisture retention properties and the like of the prolate-spheroidal polymer particles A. The upper limit of the content is preferably 99% by weight, more preferably 90% by weight, further preferably 80% by weight, and most preferably 70% by weight. Particularly preferable examples of the oily component include higher alcohols, hydrocarbons, vegetable oils and fats, and various silicones. The oily component can be used singly or in combination of two or more types.

The skin cosmetic of the present invention can include, as other components other than the components (B) and (C), additional components such as organic powders; mineral salts; flavors; dyes; colorants; pigments; various surfactants such as nonionic surfactants, anionic surfactants, cationic surfactants, and amphoteric surfactants; alcohols such as lower alcohols, polyhydric alcohols, sugars, and sterols; solvents; propellants; polymers; thickeners; gelling agents; UV absorbers; antioxidants; reducing agents; oxidizing agents; moisturizers; feel improvers; sequestering agents; antiseptics; antibacterial agents; animal and plant extracts; pH adjusters; acids/alkalis; whitening agents; vitamins and derivatives thereof; antiphlogistics; anti-inflammatory agents; hair restorers; blood circulation promoting agents; stimulants; hormones; anti-wrinkle agents; anti-aging agents; tightening agents; cooling agents; warming agents; wound healing promoting agents; stimulation reducing agents; analgesic agents; cell activating agents; antipruritic agents; exfoliating agents; solubilizers; antiperspirants; refrigerants; enzymes; nucleic acid; and water. These components can be appropriately compounded to the extent that the effect of the present invention is not impaired. These components can be used singly or in combination of two or more types.

In addition to those described above, the skin cosmetic of the present invention can contain known components such as components described in Japan Cosmetic Industry Association component display name list, INCI Dictionary (The International Cosmetic Ingredient Dictionary and Handbook), Quasi-drug raw material standard (including cosmetic raw material standard [old] and cosmetic compounding component standard by category [old]), Pharmacopeia of Japan, Pharmaceutical Additives Standard, Food Additives Official Compendium and the like, and components described in Japanese and foreign patents and patent publications (including Japanese Translation of PCT International Application Publication/republication) that belong to the A61K8 and C11D classifications of the International Patent Classification (IPC), in known combinations, compounding ratios, and compounding amounts.

Examples of the skin cosmetic of the present invention include skin care products, hair products, antiperspirant products, makeup products, UV protection products, and perfume products. More specifically, examples of the skin cosmetic of the present invention include basic cosmetics such as milky lotions, creams, lotions, calamine lotions, sunscreen agents, makeup bases, suntan agents, after-shave lotions, pre-shave lotions, packs, cleansing agents, face washes, acne control cosmetics, and essences, makeup cosmetics such as foundations, face powders, mascaras, concealers, eye shadows, eyeliners, eyebrow pencils, cheek rouges, nail polishes, lip creams, and lipsticks, shampoos, rinses, conditioners, hair colorings, hair tonics, setting agents, body powders, hair restorers, deodorants, depilatories, soaps, body shampoos, bath additives, hand soaps, and perfumes. The form of the product is not particularly limited, and examples of the form include various forms such as liquid, emulsion, cream, solid, paste, gel, powder, multilayer, mousse, and spray.

## EXAMPLES

Hereinafter, though the present invention is more specifically described by way of Production Examples, Reference Examples, Examples, and Comparative Examples, the present invention is not limited to thereto. The evaluation items in each Production Example and Comparative Production Example below were evaluated by the following method.

(1) Volume mean particle size, number mean particle size, and dispersion range of polymer particles

The measurement was performed using a laser diffraction/scattering type particle size distribution measurement device MICROTRACK MT3300EX-II (manufactured by MicrotracBEL Corp.).

(2) Aspect ratio of polymer particles

For the prolate-spheroidal polymer particles, using a scanning electron microscope (S-4800 manufactured by Hitachi High-Technologies Corporation, hereinafter referred to as SEM), the photograph of the particles was taken at a measurable magnification (300 to 30,000 times), one hundred particles were randomly extracted in a state where the obtained prolate-spheroidal polymer particles were made two-dimensional (usually, the prolate-spheroidal polymer particles are in a state where the major axis direction of the prolate-spheroidal polymer particles are kept horizontal), the long diameter ($L^1$) and the short diameter ($D^1$) of each particle were measured, the aspect ratio ($L^1/D^1$) was calculated, and the average of the aspect ratio ($P^1_{AV}$) was calculated.

Similarly, the long diameter ($L^1$) and the short diameter ($D^1$) of 100 randomly extracted particles were measured and the average of the long diameter ($L^1_{AV}$) and the average of the short diameter ($D^1_{AV}$) of the particles were calculated.

For the flat ellipsoidal polymer particles, similarly, the photograph of the particles was taken with SEM, one hundred particles were randomly extracted in a state where the obtained flat ellipsoidal polymer particles were made two-dimensional, the long diameter ($L^2$) of the flat part, the short diameter ($D^2$) of the flat part, and the thickness ($T^2$) of the side of each particle were measured, and
the average of aspect ratio ($L^2/D^2$)($P^2_{AV}$),
the average of aspect ratio ($D^2/T^2$)($P^3_{AV}$), and
the average of aspect ratio ($L^2/T^2$)($P^4_{AV}$)

were calculated.

Similarly, the long diameter ($L^2$), the short diameter ($D^2$), and the thickness ($T^2$) of the side of 100 randomly extracted particles were measured and the average of the long diameter ($L^2_{AV}$), the average of the short diameter ($D^2_{AV}$), and the average of the thickness ($T^2_{AV}$) of the flat part of the particles were calculated. FIG. 13 is a diagram showing the long diameter ($L^2$), the short diameter ($D^2$), and the thickness ($T^2$) of flat ellipsoidal polymer particles.

(3) Bulk density of polymer particles

The bulk density was calculated as loose bulk density using the 1st method (the method in which a measuring cylinder is used) of the test method defined by three pharmacopoeias. The unit was g/mL.

(4) Measurement of water absorption

The dried polymer particle powder was dispersed in water at a concentration of about 2% by weight, allowed to stand for one day, then dispersed again, and filtered under reduced pressure using a glass filter. The glass filter used for the filtration was centrifuged at 3,000 rpm for 30 minutes using a centrifuge (CR-20GII manufactured by Hitachi High-Technologies Corporation), the obtained polymer particle powder was dried, the weights of the powder before and after drying were measured, and the difference between the weights was taken as water absorption.

(5) Measurement of oil absorption

The oil absorption was measured according to the Boiled linseed oil method described in JIS K 5101.

(6) pH measurement

pH was measured by immersing a pH test paper (manufactured by Whatman) in a reaction liquid being stirred and observing the change degree of the color.

(7) Softening temperature

Dried polymer particle powder (10 mg) was put in an aluminum pan, using an empty aluminum pan as a reference, the measurement was performed under conditions of the measurement temperature range of 25 to 250°C and the rate of temperature rise of 20°C/min using a differential scanning calorimeter (DSC 6200 manufactured by Hitachi High-Tech Science Corporation), and the temperature at which the endothermic peak was shown in the DSC curve was taken as the softening temperature.

[1] Production of polymer particles

[Production Example 1] Production of polymer particles A1

The compounds shown below were put at once to a 2,000 mL flask to prepare a synthesis solution. Then, the solution was stirred at room temperature for 1 hour. The liquid phase was in a state where the aqueous phase part, the emulsion phase part, and the oil phase part were mixed. Next, the temperature of an oil bath was set at 80°C, heating and stirring (350 rpm) of the solution was started under nitrogen stream, and the polymerization reaction was performed for 8 hours to obtain a methyl methacrylate-ethylene glycol dimethacrylate copolymer particle dispersion. The pH of the reaction liquid before the start of heating was 7, the pH of the reaction liquid 2 hours after the start of heating was 2, and the pH of the reaction liquid at the end of the reaction was 1.

| | |
|---|---|
| Water | 1250.0 g |
| Ethanol | 86.5 g |
| Polypropylene glycol (# 3000) | 35.0 g |
| Polyvinyl pyrrolidone (K-15) | 43.5 g |
| Sucrose laurate ester | 8.5 g |
| Ammonium persulfate | 22.0 g |
| Methyl methacrylate | 365.5 g |
| Ethylene glycol dimethacrylate | 2.9 g |

The obtained particle dispersion was passed through a 200 μm sieve into a separate 3,000 mL flask. Next, using known suction filtration equipment, the particle dispersion that was passed through the sieve was washed and filtered 5 times with methanol, and vacuum dried, thereby polymer particles A1 were obtained. The compositional ratio (molar ratio) of each repeating unit in the polymer particles A1 was methyl methacrylate : ethylene glycol dimethacrylate = 99 : 1. One hundred of the obtained particles were randomly extracted, and their shapes were observed by SEM, and were found to be spherical particles having $L^1_{AV}$ of 14.5 μm, $D^1_{AV}$ of 5.9 μm, and $P^1_{AV}$ of 2.5. The polymer particles A1 had fine irregularities formed by binding or containing particles having an average particle size of 0.4 μm or more in the surface layer and on the surface. The volume mean particle size ($MV^1$) of the polymer particles A1 was 7.1 μm. The SEM photographs of the polymer particles A1 are shown in FIG. 1 and FIG. 2.

[Production Example 2] Production of polymer particles A2

The compounds shown below were put at once to a 2,000 mL flask to prepare a synthesis solution. Then, the solution was stirred at room temperature for 1 hour. The liquid phase was in a state where the aqueous phase part, the emulsion phase part, and the oil phase part were mixed. Next, the temperature of an oil bath was set at 76°C, heating and stirring (350 rpm) of the solution was started under nitrogen stream, and the polymerization reaction was performed for 8 hours to obtain a methyl methacrylate-ethylene glycol dimethacrylate copolymer particle dispersion. The pH of the reaction liquid before the start of heating was 7, the pH of the reaction liquid 2 hours after the start of heating was 2, and the pH of the reaction liquid at the end of the reaction was 1.

| | |
|---|---|
| Water | 1250.0 g |
| Ethanol | 86.5 g |
| Polypropylene glycol (# 3000) | 22.5 g |
| Polyvinyl pyrrolidone (K-15) | 43.5 g |
| Sucrose laurate ester | 6.5 g |
| Ammonium persulfate | 22.0 g |
| Methyl methacrylate | 365.5 g |
| Ethylene glycol dimethacrylate | 2.9 g |

The obtained particle dispersion was passed through a 200 μm sieve into a separate 3,000 mL flask. Next, using known suction filtration equipment, the particle dispersion that was passed through the sieve was washed and filtered 5 times with methanol, and vacuum dried, thereby polymer particles A2 were obtained. The compositional ratio (molar ratio) of each repeating unit in the polymer particles A2 was methyl methacrylate : ethylene glycol dimethacrylate = 99 : 1. One hundred of the obtained particles were randomly extracted, and their shapes were observed by SEM. It was found that the polymer particles A2 are spherical particles having $L^1_{AV}$ of 20.7 μm, $D^1_{AV}$ of 10.3 μm, and $P^1_{AV}$ of 2.0. The polymer particles A2 had fine irregularities formed by binding or containing particles having an average particle size of 0.6 μm or more in the surface layer and on the surface. The $MV^1$ of the polymer particles A2 was 14.5 μm. The SEM photographs of the polymer particles A2 are shown in FIG. 3 and FIG. 4.

[Production Example 3] Production of polymer particles A3

The compounds shown below were put at once to a 2,000 mL flask to prepare a synthesis solution. Then, the solution was stirred at room temperature for 1 hour. The liquid phase was in a state where the aqueous phase part, the emulsion phase part, and the oil phase part were mixed. Next, the temperature of an oil bath was set at 76°C, heating and stirring (300 rpm) of the solution was started under nitrogen stream, and the polymerization reaction was performed for 8 hours to obtain a methyl methacrylate-ethylene glycol dimethacrylate copolymer particle dispersion. The pH of the reaction liquid before the start of heating was 7, the pH of the reaction liquid 2 hours after the start of heating was 2, and the pH of the reaction liquid at the end of the reaction was 1.

| | |
|---|---|
| Water | 1295.0 g |
| Ethanol | 60.5 g |
| Polypropylene glycol (# 3000) | 25.5 g |
| Polyvinyl pyrrolidone (K-15) | 30.8 g |
| Sucrose laurate ester | 5.5 g |
| Ammonium persulfate | 30.4 g |
| Methyl methacrylate | 380.0 g |

(continued)

| | |
|---|---|
| Ethylene glycol dimethacrylate | 1.9 g |

The obtained particle dispersion was passed through a 200 μm sieve into a separate 3,000 mL flask. Next, using known suction filtration equipment, the particle dispersion that was passed through the sieve was washed and filtered 5 times with methanol, and vacuum dried, thereby polymer particles A3 were obtained. The compositional ratio (molar ratio) of each repeating unit in the polymer particles A3 was methyl methacrylate : ethylene glycol dimethacrylate = 99 : 1. One hundred of the obtained particles were randomly extracted, and their shapes were observed by SEM. It was found that the polymer particles A3 are spherical particles having $L^1_{AV}$ of 32.6 μm, $D^1_{AV}$ of 18.1 μm, and $P^1_{AV}$ of 1.8. The polymer particles A3 had fine irregularities formed by binding or containing particles having an average particle size of 1.2 μm or more in the surface layer and on the surface. The $MV^1$ of the polymer particles A3 was 21.8 μm. The SEM photographs of the polymer particles A3 are shown in FIG. 5 and FIG. 6.

[Production Example 4] Production of polymer particles A4

The compounds shown below were put at once to a 2,000 mL flask to prepare a synthesis solution. Then, the solution was stirred at room temperature for 1 hour. The liquid phase was in a state where the aqueous phase part, the emulsion phase part, and the oil phase part were mixed. Next, the temperature of an oil bath was set at 80°C, heating and stirring (400 rpm) of the solution was started under nitrogen stream, and the polymerization reaction was performed for 8 hours to obtain a methyl methacrylate-ethylene glycol dimethacrylate copolymer particle dispersion. The pH of the reaction liquid before the start of heating was 7, the pH of the reaction liquid 2 hours after the start of heating was 3, and the pH of the reaction liquid at the end of the reaction was 1.

| | |
|---|---|
| Water | 1230.0 g |
| Ethanol | 115.5 g |
| Polypropylene glycol (# 3000) | 43.5 g |
| Polyvinyl pyrrolidone (K-15) | 44.0 g |
| Sucrose laurate ester | 11.2 g |
| Ammonium persulfate | 27.6 g |
| Methyl methacrylate | 345.0 g |
| Ethylene glycol dimethacrylate | 1.7 g |

The obtained particle dispersion was passed through a 200 μm sieve into a separate 3,000 mL flask. Next, using known suction filtration equipment, the particle dispersion that was passed through the sieve was washed and filtered 5 times with methanol, and vacuum dried, thereby polymer particles A4 were obtained. The compositional ratio (molar ratio) of each repeating unit in the polymer particles A4 was methyl methacrylate : ethylene glycol dimethacrylate = 99 : 1. One hundred of the obtained particles were randomly extracted, and their shapes were observed by SEM. It was found that the polymer particles A4 are spherical particles having $L^1_{AV}$ of 48.8 μm, $D^1_{AV}$ of 3.2 μm, and $P^1_{AV}$ of 15.2. The polymer particles A4 had fine irregularities formed by binding or containing particles having an average particle size of 0.3 μm or more in the surface layer and on the surface. The $MV^1$ of the polymer particles A4 was 8.9 μm. The SEM photographs of the polymer particles A4 are shown in FIG. 7 and FIG. 8.

[Production Example 5] Production of polymer particles A5

The compounds shown below were put at once to a 2,000 mL flask to prepare a synthesis solution. Then, the solution was stirred at room temperature for 1 hour. The liquid phase was in a state where the aqueous phase part, the emulsion phase part, and the oil phase part were mixed. Next, the temperature of an oil bath was set at 80°C, heating and stirring (400 rpm) of the solution was started under nitrogen stream, and the polymerization reaction was performed for 8 hours to obtain a methyl methacrylate-ethylene glycol dimethacrylate copolymer particle dispersion. The pH of the reaction liquid before the start of heating was 7, the pH of the reaction liquid 2 hours after the start of heating was 3, and the pH of the reaction liquid at the end of the reaction was 1.

| | |
|---|---|
| Water | 1215.0 g |
| Ethanol | 92.5 g |
| Polypropylene glycol (# 3000) | 40.0 g |

(continued)

| | |
|---|---|
| Polyvinyl pyrrolidone (K-15) | 48.0 g |
| Sucrose laurate ester | 10.5 g |
| Ammonium persulfate | 27.6 g |
| Methyl methacrylate | 375.0 g |
| Ethylene glycol dimethacrylate | 2.3 g |

The obtained particle dispersion was passed through a 200 μm sieve into a separate 3,000 mL flask. Next, using known suction filtration equipment, the particle dispersion that was passed through the sieve was washed and filtered 5 times with methanol, and vacuum dried, thereby polymer particles A5 were obtained. The compositional ratio (molar ratio) of each repeating unit in the polymer particles A5 was methyl methacrylate : ethylene glycol dimethacrylate = 99 : 1. One hundred of the obtained particles were randomly extracted, and their shapes were observed by SEM. It was found that the polymer particles A5 are spherical particles having $L^1_{AV}$ of 20.4 μm, $D^1_{AV}$ of 3.0 μm, and $P^1_{AV}$ of 6.9. The polymer particles A5 had fine irregularities formed by binding or containing particles having an average particle size of 0.4 μm or more in the surface layer and on the surface. The $MV^1$ of the polymer particles A5 was 6.6 μm. The SEM photographs of the polymer particles A5 are shown in FIG. 9 and FIG. 10.

[Production Example 6] Production of polymer particles A6

The compounds shown below were put at once to a 2,000 mL flask to prepare a synthesis solution. Then, the solution was stirred at room temperature for 1 hour. The liquid phase was in a state where the aqueous phase part, the emulsion phase part, and the oil phase part were mixed. Next, the temperature of an oil bath was set at 80°C, heating and stirring (400 rpm) of the solution was started under nitrogen stream, and the polymerization reaction was performed for 8 hours to obtain a methyl methacrylate-ethylene glycol dimethacrylate copolymer particle dispersion. The pH of the reaction liquid before the start of heating was 7, the pH of the reaction liquid 2 hours after the start of heating was 2, and the pH of the reaction liquid at the end of the reaction was 1.

| | |
|---|---|
| Water | 1210.0 g |
| Ethanol | 105.0 g |
| Polypropylene glycol (# 3000) | 42.0 g |
| Polyvinyl pyrrolidone (K-15) | 68.5 g |
| Sucrose laurate ester | 12.0 g |
| Ammonium persulfate | 28.4 g |
| Methyl methacrylate | 355.0 g |
| Ethylene glycol dimethacrylate | 2.8 g |

The obtained particle dispersion was passed through a 200 μm sieve into a separate 3,000 mL flask. Next, using known suction filtration equipment, the particle dispersion that was passed through the sieve was washed and filtered 5 times with methanol, and vacuum dried, thereby polymer particles A6 were obtained. The compositional ratio (molar ratio) of each repeating unit in the polymer particles A6 was methyl methacrylate : ethylene glycol dimethacrylate = 98 : 2. One hundred of the obtained particles were randomly extracted, and their shapes were observed by SEM. It was found that the polymer particles A6 are spherical particles having $L^1_{AV}$ of 14.2 μm, $D^1_{AV}$ of 1.3 μm, and $P^1_{AV}$ of 10.9. The polymer particles A6 had fine irregularities formed by binding or containing particles having an average particle size of 0.2 μm or more in the surface layer and on the surface. The $MV^1$ of the polymer particles A6 was 3.7 μm. The SEM photographs of the polymer particles A6 are shown in FIG. 11 and FIG. 12.

[Production Example 7] Production of polymer particles A7

Polymer particles A7 were produced in the same manner as in Production Example 4 except that the amount of the sucrose laurate ester was changed to 14.5 g. The pH of the reaction liquid before the start of heating was 7, the pH of the reaction liquid 2 hours after the start of heating was 3, and the pH of the reaction liquid at the end of the reaction was 1.

The compositional ratio (molar ratio) of each repeating unit in the polymer particles A7 was methyl methacrylate : ethylene glycol dimethacrylate = 99 : 1. One hundred of the obtained particles were randomly extracted, and their shapes were observed by SEM. It was found that the polymer particles A7 are spherical particles having $L^1_{AV}$ of 50.2 μm, $D^1_{AV}$

of 10.3 μm, and $P^1_{AV}$ of 4.9. The polymer particles A7 had fine irregularities formed by binding or containing particles having an average particle size of 1.4 μm or more in the surface layer and on the surface. The $MV^1$ of the polymer particles A7 was 21.3 μm.

[Production Example 8] Production of polymer particles A8

Polymer particles A8 were produced in the same manner as in Production Example 1 except that methyl methacrylate was changed to styrene and ethylene glycol dimethacrylate was changed to divinylbenzene. The pH of the reaction liquid before the start of heating was 7, the pH of the reaction liquid 2 hours after the start of heating was 2, and the pH of the reaction liquid at the end of the reaction was 1.

The compositional ratio (molar ratio) of each repeating unit in the polymer particles A8 was styrene : divinylbenzene = 99 : 1. One hundred of the obtained particles were randomly extracted, and their shapes were observed by SEM. It was found that the polymer particles A8 are spherical particles having $L^1_{AV}$ of 12.5 μm, $D^1_{AV}$ of 4.0 μm, and $P^1_{AV}$ of 3.1. The polymer particles A8 had fine irregularities formed by binding or containing particles having an average particle size of 0.8 μm or more in the surface layer and on the surface. The $MV^1$ of the polymer particles A8 was 5.6 μm.

[Production Example 9] Production of polymer particles A9

The compounds shown below were put at once to a 2,000 mL flask to prepare a synthesis solution. Then, the solution was stirred at room temperature for 1 hour. The liquid phase was in a state where the aqueous phase part, the emulsion phase part, and the oil phase part were mixed. The temperature of an oil bath was set at 76°C, heating and stirring (350 rpm) of the solution was started under nitrogen stream, and the polymerization reaction was performed for 8 hours to obtain a styrene : 2-HEMA : divinylbenzene copolymer particle dispersion. When the internal temperature reached 40°C, 1 mol/L hydrochloric acid was added dropwise to the synthesis solution as a pH adjuster over 15 minutes until the pH reached 2. The pH at the end of the reaction was also 2.

| | |
|---|---|
| Water | 913.0 g |
| Ethanol | 392.0 g |
| Polypropylene glycol (# 3000) | 22.5 g |
| Polyvinyl pyrrolidone (K-15) | 50.0 g |
| Sucrose laurate ester | 8.0 g |
| Azobisisobutyronitrile | 15.0 g |
| Styrene | 195.0 g |
| 2-hydroxyethyl methacrylate (2-HEMA) | 87.0 g |
| Divinylbenzene | 3.0 g |

The obtained particle dispersion was passed through a 200 μm sieve into a separate 3,000 mL flask. Next, using known suction filtration equipment, the particle dispersion that was passed through the sieve was washed and filtered 5 times with methanol, and vacuum dried, thereby polymer particles A9 were obtained. The compositional ratio (molar ratio) of each repeating unit in the polymer particles A9 was styrene : 2-HEMA : divinylbenzene = 64 : 35 : 1. One hundred of the obtained particles were randomly extracted, and their shapes were observed by SEM. It was found that the polymer particles A9 are spherical particles having $L^1_{AV}$ of 10.2 μm, $D^1_{AV}$ of 5.1 μm, and $P^1_{AV}$ of 2.0. The polymer particles A9 had fine irregularities formed by binding or containing particles having an average particle size of 1.1 μm or more in the surface layer and on the surface. The $MV^1$ of the polymer particles A9 was 6.0 μm.

[Production Example 10] Production of polymer particles A10

The compounds shown below were put at once to a 2,000 mL flask to prepare a synthesis solution. Then, the solution was stirred at room temperature for 1 hour. The liquid phase was in a state where the aqueous phase part, the emulsion phase part, and the oil phase part were mixed. Next, the temperature of an oil bath was set at 80°C, heating and stirring (400 rpm) of the solution was started under nitrogen stream, and the polymerization reaction was performed for 8 hours to obtain a styrene : methacrylic acid : ethylene glycol dimethacrylate copolymer particle dispersion. When the internal temperature reached 40°C, 1 mol/L hydrochloric acid was added dropwise to the synthesis solution as a pH adjuster over 15 minutes until the pH reached 2. The pH at the end of the reaction was also 2.

| | |
|---|---|
| Water | 440.0 g |

(continued)

| | |
|---|---|
| Ethanol | 780.0 g |
| Polypropylene glycol (# 3000) | 15.5 g |
| Polyvinyl pyrrolidone (K-15) | 67.5 g |
| Sucrose laurate ester | 10.0 g |
| Azobisisobutyronitrile | 9.5 g |
| Styrene | 202.5 g |
| Methacrylic acid | 62.1 g |
| Ethylene glycol dimethacrylate | 5.4 g |

The obtained particle dispersion was passed through a 200 μm sieve into a separate 3,000 mL flask. Next, using known suction filtration equipment, the particle dispersion that was passed through the sieve was washed and filtered 5 times with methanol, and vacuum dried, thereby polymer particles A10 were obtained. The compositional ratio (molar ratio) of each repeating unit in the polymer particles A10 was styrene : methacrylic acid : ethylene glycol dimethacrylate = 77 : 19 : 4. One hundred of the obtained particles were randomly extracted, and their shapes were observed by SEM. It was found that the polymer particles A10 are spherical particles having $L^1_{AV}$ of 8.1 μm, $D^1_{AV}$ of 1.5 μm, and $P^1_{AV}$ of 5.4.
The polymer particles A10 had fine irregularities formed by binding or containing particles having an average particle size of 0.4 μm or more in the surface layer and on the surface. The $MV^1$ of the polymer particles A10 was 2.9 μm.

[Production Example 11] Production of polymer particles B1

The components shown below were put at once to a 2,000 mL flask and stirred at room temperature for 1 hour. At this time, the liquid phase was in a state where the aqueous phase part, the emulsion phase part, and the oil phase part were mixed.

| | |
|---|---|
| Water | 890.0 g |
| Ethanol | 55.5 g |
| Polypropylene glycol (# 2000) | 88.0 g |
| Polyvinyl pyrrolidone (K-15) | 40.5 g |
| Sucrose laurate ester | 12.5 g |
| Dimethyl 2,2'-azobis(isobutyrate) | 3.3 g |
| Ammonium persulfate | 9.5 g |
| Methyl methacrylate | 655.0 g |
| Ethylene glycol dimethacrylate | 6.6 g |

Next, the temperature of an oil bath was set at 75°C, heating and stirring (400 rpm) of the solution was started under nitrogen stream, and the polymerization reaction was performed for 8 hours to obtain a methyl methacrylate-ethylene glycol dimethacrylate copolymer particle dispersion. The pH of the reaction liquid before the start of heating was 7, the pH of the reaction liquid 2 hours after the start of heating was 3, and the pH of the reaction liquid at the end of the reaction was 2.

The obtained particle dispersion was filtered using known suction filtration equipment, and the obtained filtrate was further washed and filtered 5 times with methanol, and then vacuum dried to obtain polymer particles B1. The compositional ratio (molar ratio) of each repeating unit in the polymer particles B1 was methyl methacrylate : ethylene glycol dimethacrylate = 98 : 2. One hundred of the obtained particles were randomly extracted, and their shapes were observed by SEM. It was found that the polymer particles B1 are flat ellipsoidal polymer particles having $L^2_{AV}$ of 43.3 μm, $D^2_{AV}$ of 4.2 μm, $T^2_{AV}$ of 2.0 μm, $P^2_{AV}$ of 10.3 μm, $P^3_{AV}$ of 10.3 μm, and $P^4_{AV}$ of 21.6 μm. The polymer particles B1 were flat ellipsoidal polymer particles in which particles having an average particle size of 1.9 μm or more are attached on the surface layer and the surface of the flat ellipsoidal polymer particles. The volume mean particle size ($MV^2$) of the polymer particles B1 was 19.2 μm.

[Production Example 12] Production of polymer particles B2

The polymer particles B2 were produced in the same manner as in Production Example 11 except that the components necessary for the production were changed as follows, and the temperature of an oil bath was changed to 73°C, and the stirring was performed at 300 rpm:

| | |
|---|---|
| Water | 850.0 g |
| Ethanol | 60.0 g |
| Polypropylene glycol (# 3000) | 102.5 g |
| Polyvinyl pyrrolidone (K-15) | 53.0 g |
| Sucrose laurate ester | 15.5 g |
| Azobisisobutyronitrile | 2.0 g |
| Ammonium persulfate | 5.1 g |
| Methyl methacrylate | 642.0 g |
| Ethylene glycol dimethacrylate | 12.8 g |

The pH of the reaction liquid before the start of heating was 7, the pH of the reaction liquid 2 hours after the start of heating was 3, and the pH of the reaction liquid at the end of the reaction was 2.

The compositional ratio (molar ratio) of each repeating unit in the polymer particles B2 was methyl methacrylate : ethylene glycol dimethacrylate = 96 : 4. One hundred of the obtained particles were randomly extracted, and their shapes were observed by SEM. It was found that the polymer particles B2 are flat ellipsoidal polymer particles having $L^2_{AV}$ of 24.2 μm, $D^2_{AV}$ of 10.1 μm, $T^2_{AV}$ of 4.2 μm, $P^2_{AV}$ of 2.4 μm, $P^3_{AV}$ of 2.4 μm, and $P^4_{AV}$ of 5.8 μm. The polymer particles B2 were flat ellipsoidal polymer particles in which particles having an average particle size of 1.1 μm or more are attached on the surface layer and the surface of the flat ellipsoidal polymer particles. The $MV^2$ of the polymer particles B2 was 13.1 μm.

[Production Example 13] Production of polymer particles B3

Based on the method described in Comparative Example 4 of WO 2016/181878, acrylic spherical polymer particles B3 (average particle size: 5 μm, aspect ratio: 1) including polymethyl methacrylate were produced. The polymer particles B3 did not have fine irregularities on the surface.

[Production Example 14] Production of polymer particles B4

The compounds shown below were put at once to a 2,000 mL flask, a suspension was produced with a disperion dispersing blade at 1,000 rpm, and the suspension was heated and stirred for 8 hours at an oil bath temperature of 80°C under nitrogen stream to obtain polymethyl methacrylate particle dispersion.

| | |
|---|---|
| Water | 1280.0 g |
| Styrene | 288.0 g |
| Lauryl peroxide | 14.2 g |
| Polyvinyl pyrrolidone (K-30) | 21.6 g |

The obtained particle dispersion was passed through a 200 μm sieve into a separate 3,000 mL flask. Next, the particle dispersion that was passed through the sieve was centrifuged 5 times, and classified and washed to obtain spherical polymer particles B4 of polystyrene only having an average particle size of 10 μm and an aspect ratio of 1. The polymer particles B4 did not have fine irregularities on the surface.

The bulk density, water absorption, oil absorption, and softening temperature of polymer particles A1 to A10 and B1 to B4 are shown in Table 1.

[Table 1]

| Polymer particles | Bulk density (g/mL) | Water absorption (g/100 g) | Oil absorption (g/100 g) | Softening temperature (°C) |
|---|---|---|---|---|
| A1 | 0.44 | 70.6 | 95.3 | 162.5 |
| A2 | 0.47 | 65.3 | 79.6 | 153.6 |
| A3 | 0.56 | 61.7 | 76.7 | 148.3 |
| A4 | 0.18 | 100.1 | 119.5 | 167.5 |

(continued)

| Polymer particles | Bulk density (g/mL) | Water absorption (g/100 g) | Oil absorption (g/100 g) | Softening temperature (°C) |
|---|---|---|---|---|
| A5 | 0.24 | 73.9 | 94.9 | 171.2 |
| A6 | 0.12 | 69.7 | 82.4 | 181.1 |
| A7 | 0.52 | 92.5 | 108.8 | 141.7 |
| A8 | 0.29 | 57.5 | 88.3 | 176.3 |
| A9 | 0.42 | 102.3 | 108.4 | 156.1 |
| A10 | 0.16 | 94.3 | 102.7 | 169.7 |
| B1 | 0.51 | 79.4 | 84.5 | 153.2 |
| B2 | 0.37 | 100.5 | 106.2 | 148.4 |
| B3 | 0.72 | 48.0 | 53.9 | 95.1 |
| B4 | 0.77 | 30.4 | 52.8 | 103.2 |

[2] Evaluation of polymer particles

[Evaluation test 1] Sensory test and adhesion evaluation

[Reference Examples 1-1 to 1-19, Comparative Reference Examples 1-1 to 1-2]

The polymer particles A1 to A10 and B1 to B4 were evaluated for touch, slipperiness, and particle adhesion according to the following method.

Each type of polymer particles was evaluated alone in Reference Examples 1-1 to 1-10 and Comparative Reference Examples 1-1 to 1-2, and the combination of polymer particles described in Table 2 was evaluated in Reference Examples 1-11 to 1-19. The volume mean particle size (MV), the number mean particle size (MN), the dispersion range (SD), and the average aspect ratio ($P_{AV}$) of each Reference Example and Comparative Reference Examples are shown in Table 2, and the evaluation results are shown in Table 3. The MV and $P_{AV}$ of the polymer particles A1 to A10 are the same as the above-mentioned $MV^1$ and $P^1_{AV}$, respectively, and the MV and $P_{AV}$ of the polymer particles B3 to B4 are the same as the above-mentioned mean particle size and aspect ratio, respectively.

(1) Touch

The feeling when each type of particles was spread on the skin was evaluated according to the following criteria.

(2) Slipperiness

The length when each type of particles (1 g) was placed on a black artificial leather, and spread with fingers was evaluated according to the following criteria.
◎: Excellent, ○: Good, Δ: Fair, ×: Bad

(3) Particle adhesion

Each type of particles (1 g) was placed on a black artificial leather, and spread evenly with a puff, then the artificial leather was tapped three times, and the remaining amount of the particles was observed with a digital microscope (VHX200 manufactured by KEYENCE CORPORATION) to evaluate the particle adhesion with the following criteria.
◎: Excellent, ○: Good, Δ: Fair, ×: Bad

(4) Light diffusibility/gloss

Each type of particles was spread on the skin, and irradiated with fluorescent light from all directions to evaluate the light diffusibility and gloss.
◎: Excellent, ○: Good, Δ: Fair, ×: Bad

[Table 2]

| | | Polymer particles | MV (μm) | MN (μm) | MV-MN (μm) | $P_{AV}$ | SD (μm) | Condition (12) | Condition (13) | Condition (14) |
|---|---|---|---|---|---|---|---|---|---|---|
| Reference Example | 1-1 | A1 | 7.1 | 5.2 | 1.9 | 2.5 | 2.4 | - | - | - |
| | 1-2 | A2 | 14.5 | 11.2 | 3.3 | 2.0 | 2.9 | - | - | - |
| | 1-3 | A3 | 18.9 | 13.5 | 5.4 | 1.8 | 5.8 | - | - | - |
| | 1-4 | A4 | 8.9 | 6.0 | 2.9 | 15.2 | 2.9 | - | - | - |
| | 1-5 | A5 | 6.6 | 4.0 | 2.6 | 6.9 | 2.8 | - | - | - |
| | 1-6 | A6 | 3.7 | 2.2 | 1.5 | 10.9 | 2.1 | - | - | - |
| | 1-7 | A7 | 21.3 | 14.5 | 6.8 | 4.9 | 4.6 | - | - | - |
| | 1-8 | A8 | 5.6 | 3.6 | 2.0 | 3.1 | 2.4 | - | - | - |
| | 1-9 | A9 | 6.0 | 4.2 | 1.8 | 2.0 | 2.7 | - | - | - |
| | 1-10 | A10 | 2.9 | 1.8 | 1.1 | 5.4 | 2.2 | - | - | - |
| | 1-11 | A1 + A2 (1:1 (weight ratio)) | 10.9 | 5.1 | 5.8 | Maximum A1 Minimum A2 | 3.7 | × | ○ | × |
| | 1-12 | A1 + A4 (1:1 (weight ratio)) | 8.4 | 3.7 | 4.7 | Maximum A4 Minimum A1 | 4.6 | ○ | × | ○ |
| | 1-13 | A4 + A5 (1:1 (weight ratio)) | 7.4 | 3.9 | 3.5 | Maximum A4 Minimum A5 | 4.3 | ○ | × | × |
| | 1-14 | A4 + A6 (1:1 (weight ratio)) | 6.4 | 3.0 | 3.4 | Maximum A4 Minimum A6 | 3.7 | ○ | ○ | × |
| | 1-15 | A2 + A4 + A6 (1:1:1 (weight ratio)) | 8.9 | 3.0 | 5.9 | MaximumA4 Minimum A2 | 5.6 | ○ | ○ | ○ |
| | 1-16 | A4 + A5 + A6 (1:1:1 (weight ratio)) | 7.0 | 3.6 | 3.4 | MaximumA4 Minimum A6 | 3.9 | ○ | ○ | ○ |
| | 1-17 | A1 + A3 + B1 (1:1:1 (weight ratio)) | 12.8 | 5.1 | 7.7 | Maximum B1 Minimum A3 | 7.1 | ○ | ○ | ○ |
| | 1-18 | A4 + A5 + B2 (1:1:1 (weight ratio)) | 9.1 | 3.4 | 5.7 | MaximumA4 Minimum B2 | 5.3 | ○ | ○ | ○ |

(continued)

| | | Polymer particles | MV (μm) | MN (μm) | MV-MN (μm) | $P_{AV}$ | SD (μm) | Condition (12) | Condition (13) | Condition (14) |
|---|---|---|---|---|---|---|---|---|---|---|
| | 1-19 | A4 + A6 + B3 (2:2:1 (weight ratio)) | 6.3 | 3.1 | 3.2 | Maximum A4 Minimum A6 | 3.9 | ○ | ○ | ○ |
| Comparative Reference Example | 1-1 | B3 | 5.0 | 3.6 | 1.4 | 1 | 1.3 | - | - | - |
| | 1-2 | B4 | 10.0 | 5.1 | 4.9 | 1 | 2.4 | - | - | - |

In the table, conditions (12), (13), and (14) are as follows (the same applies hereinafter).

(12) a difference between a maximum and a minimum of the average ($P^2_{AV}$ in the case of the flat ellipsoidal polymer particles) of an aspect ratio of two or more;

(13) a difference between a maximum and a minimum of a volume mean particle size of 3 μm or more; and

(14) appearance of two or more peaks in particle size distribution measurement.

[Table 3]

| | | Polymer particles | Touch | Slipperiness | Particle adhesion | Light diffusibility | Gloss |
|---|---|---|---|---|---|---|---|
| Reference Example | 1-1 | A1 | ○ | ◎ | ○ | ◎ | ○ |
| | 1-2 | A2 | ◎ | ◎ | ○ | ○ | ◎ |
| | 1-3 | A3 | ○ | ○ | ○ | ○ | ◎ |
| | 1-4 | A4 | ○ | ○ | ◎ | ○ | ◎ |
| | 1-5 | A5 | ◎ | ◎ | ○ | ○ | ○ |
| | 1-6 | A6 | ○ | ○ | ◎ | ◎ | ○ |
| | 1-7 | A7 | ○ | ○ | ○ | ○ | ◎ |
| | 1-8 | A8 | ◎ | ◎ | ○ | ○ | ○ |
| | 1-9 | A9 | ○ | ◎ | ○ | ○ | ◎ |
| | 1-10 | A10 | ○ | ○ | ◎ | ◎ | ○ |
| | 1-11 | A1 + A2 (1:1 (weight ratio)) | ◎ | ◎ | ○ | ◎ | ◎ |
| | 1-12 | A1+A4 (1:1 (weight ratio)) | ○ | ◎ | ◎ | ◎ | ◎ |
| | 1-13 | A4 + A5 (1:1 (weight ratio)) | ◎ | ◎ | ◎ | ○ | ◎ |
| | 1-14 | A4 + A6 (1:1 (weight ratio)) | ○ | ○ | ◎ | ◎ | ◎ |
| | 1-15 | A2 + A4 + A6 (1:1:1 (weight ratio)) | ◎ | ◎ | ◎ | ◎ | ◎ |
| | 1-16 | A4 + A5 + A6 (1:1:1 (weight ratio)) | ◎ | ◎ | ◎ | ◎ | ◎ |
| | 1-17 | A1+ A3 + B1 (1:1:1 (weight ratio)) | ◎ | ◎ | ◎ | ◎ | ◎ |
| | 1-18 | A4 + A5 + B2 (1:1:1 (weight ratio)) | ◎ | ◎ | ◎ | ◎ | ◎ |
| | 1-19 | A4 + A6 + B3 (2:2:1 (weight ratio)) | ◎ | ◎ | ◎ | ◎ | ◎ |
| Comparative Reference Example | 1-1 | B3 | Δ | ○ | × | Δ | ○ |
| | 1-2 | B4 | Δ | ○ | × | Δ | ○ |

As shown in Table 3, it was confirmed that for the touch, the slipperiness, and the particle adhesion, the light diffusibility, and the gloss, prolate-spheroidal polymer particles were superior to spherical polymer particles. In particular, it was confirmed that the properties that are superior are uniquely different depending on each peculiar shape, and thus, composite performance is expected to be provided by selecting and mixing shapes according to the product concept. It was proved that superiority was further enhanced by compounding flat ellipsoidal polymer particles.

[Reference Examples 2-1 to 2-19, Comparative Reference Examples 2-1 to 2-2]

The polymer particles and purified water were mixed according to the compositions described in Table 4 below, to prepare polymer particle dispersions 1 to 21 of 0.05% by weight.

Each dispersion was placed in a glass 20 mL standard sample bottle, and the gradation effect of printed characters was evaluated through the glass bottle according to the following criteria.
◎: Excellent, ○: Good, Δ: Fair, ×: Bad

The results are shown in Table 4.

[Table 4]

| | | Polymer particle dispersion | Polymer particles | Addition amount of particles (g) | Purified water (g) | Gradation effect |
|---|---|---|---|---|---|---|
| Reference Example | 2-1 | 1 | A1 | 0.01 | 19.99 | ◎ |
| | 2-2 | 2 | A2 | 0.01 | 19.99 | ○ |
| | 2-3 | 3 | A3 | 0.01 | 19.99 | ○ |
| | 2-4 | 4 | A4 | 0.01 | 19.99 | ○ |
| | 2-5 | 5 | A5 | 0.01 | 19.99 | ○ |
| | 2-6 | 6 | A6 | 0.01 | 19.99 | ◎ |
| | 2-7 | 7 | A7 | 0.01 | 19.99 | ○ |
| | 2-8 | 8 | A8 | 0.01 | 19.99 | ○ |
| | 2-9 | 9 | A9 | 0.01 | 19.99 | ○ |
| | 2-10 | 10 | A10 | 0.01 | 19.99 | ◎ |
| | 2-11 | 11 | A1 + A2 (1:1 (weight ratio)) | 0.01 | 19.99 | ○ |
| | 2-12 | 12 | A1 + A4 (1:1 (weight ratio)) | 0.01 | 19.99 | ○ |
| | 2-13 | 13 | A4 + A5 (1:1 (weight ratio)) | 0.01 | 19.99 | ○ |
| | 2-14 | 14 | A4 + A6 (1:1 (weight ratio)) | 0.01 | 19.99 | ◎ |
| | 2-15 | 15 | A2 + A4 + A6 (1:1:1 (weight ratio)) | 0.01 | 19.99 | ◎ |
| | 2-16 | 16 | A4 + A5 + A6 (1:1:1 (weight ratio)) | 0.01 | 19.99 | ◎ |
| | 2-17 | 17 | A1 + A3 + B1 (1:1:1 (weight ratio)) | 0.01 | 19.99 | ◎ |
| | 2-18 | 18 | A4 + A5 + B2 (1:1:1 (weight ratio)) | 0.01 | 19.99 | ◎ |
| | 2-19 | 19 | A4 + A6 + B3 (2:2:1 (weight ratio)) | 0.01 | 19.99 | ◎ |
| Comparative Reference Example | 2-1 | 20 | B3 | 0.01 | 19.99 | Δ |
| | 2-2 | 21 | B4 | 0.01 | 19.99 | Δ |

[3] Production and evaluation of skin cosmetic

[Evaluation test 3] Powdery foundation

[Examples 1 to 8, Comparative Examples 1 to 2]

Makeup compositions (foundations 1 to 10) were produced using the polymer particles A1 to A6 and B1 to B4 according to the compositions described in Table 5 below.

The volume mean particle size (MV) and the dispersion range (SD) in the combinations of polymer particles compounded in foundations 1 to 10 were measured. The results are shown in Table 6.

[Table 5]

| | | Example | | | | | | | | Comparative Example | |
|---|---|---|---|---|---|---|---|---|---|---|---|
| | | 1 | 2 | 3 | 4 | 5 | 6 | 7 | 8 | 1 | 2 |
| | | Foundation | | | | | | | | | |
| | | 1 | 2 | 2 | 4 | 5 | 6 | 7 | 8 | 9 | 10 |
| Component (% by weight) | Red iron oxide | 0.4 | 0.4 | 0.4 | 0.4 | 0.4 | 0.4 | 0.4 | 0.4 | 0.4 | 0.4 |
| | Yellow iron oxide | 1.0 | 1.0 | 1.0 | 1.0 | 1.0 | 1.0 | 1.0 | 1.0 | 1.0 | 1.0 |
| | Black iron oxide | 0.2 | 0.2 | 0.2 | 0.2 | 0.2 | 0.2 | 0.2 | 0.2 | 0.2 | 0.2 |
| | Titanium oxide | 7.0 | 7.0 | 7.0 | 7.0 | 7.0 | 7.0 | 7.0 | 7.0 | 7.0 | 7.0 |
| | Zinc oxide | 3.0 | 3.0 | 3.0 | 3.0 | 3.0 | 3.0 | 3.0 | 3.0 | 3.0 | 3.0 |
| | Silicone treated large particle size titanium oxide | 3.0 | 3.0 | 3.0 | 3.0 | 3.0 | 3.0 | 3.0 | 3.0 | 3.0 | 3.0 |
| | Lauroyl lysine powder | 14.0 | 14.0 | 14.0 | 14.0 | 14.0 | 14.0 | 14.0 | 14.0 | 14.0 | 14.0 |
| | Mica titanium | 4.0 | 4.0 | 4.0 | 4.0 | 4.0 | 4.0 | 4.0 | 4.0 | 4.0 | 4.0 |
| | Talc | 37.97 | 37.97 | 37.97 | 37.97 | 37.97 | 37.97 | 37.97 | 37.97 | 37.97 | 37.97 |
| | Methylphenyl polysiloxane | 2.0 | 2.0 | 2.0 | 2.0 | 2.0 | 2.0 | 2.0 | 2.0 | 2.0 | 2.0 |
| | Microcrystalline cellulose | 5.0 | 5.0 | 5.0 | 5.0 | 5.0 | 5.0 | 5.0 | 5.0 | 5.0 | 5.0 |
| | Corn starch | 10.0 | 10.0 | 10.0 | 10.0 | 10.0 | 10.0 | 10.0 | 10.0 | 10.0 | 10.0 |
| | Methylparaben | 0.1 | 0.1 | 0.1 | 0.1 | 0.1 | 0.1 | 0.1 | 0.1 | 0.1 | 0.1 |
| | Sodium dehydroacetate | 0.1 | 0.1 | 0.1 | 0.1 | 0.1 | 0.1 | 0.1 | 0.1 | 0.1 | 0.1 |
| | Liquid paraffin | 1.5 | 1.5 | 1.5 | 1.5 | 1.5 | 1.5 | 1.5 | 1.5 | 1.5 | 1.5 |
| | Butylene glycol | 0.5 | 0.5 | 0.5 | 0.5 | 0.5 | 0.5 | 0.5 | 0.5 | 0.5 | 0.5 |
| | Coix lachryma-jobi extract | 0.1 | 0.1 | 0.1 | 0.1 | 0.1 | 0.1 | 0.1 | 0.1 | 0.1 | 0.1 |
| | Ginseng extract | 0.1 | 0.1 | 0.1 | 0.1 | 0.1 | 0.1 | 0.1 | 0.1 | 0.1 | 0.1 |
| | Ubiquinone | 0.03 | 0.03 | 0.03 | 0.03 | 0.03 | 0.03 | 0.03 | 0.03 | 0.03 | 0.03 |
| | Polymer particles A1 | 5.0 | 5.0 | - | - | - | - | 4.0 | - | - | - |
| | Polymer particles A2 | 5.0 | - | - | - | 3.0 | - | - | - | - | - |
| | Polymer particles A3 | - | - | - | - | - | - | 3.0 | - | - | - |
| | Polymer particles A4 | - | 5.0 | 5.0 | 4.0 | 3.0 | 3.0 | - | 3.0 | - | - |
| | Polymer particles A5 | - | - | 5.0 | - | - | 3.0 | - | 4.0 | - | - |
| | Polymer particles A6 | - | - | - | 5.0 | 4.0 | 4.0 | - | - | - | - |
| | Polymer particles B1 | - | - | - | - | - | - | 3.0 | - | - | - |
| | Polymer particles B2 | - | - | - | - | - | - | - | 3.0 | - | - |
| | Polymer particles B3 | - | - | - | 1.0 | - | - | - | - | 10.0 | - |
| | Polymer particles B4 | - | - | - | - | - | - | - | - | - | 10.0 |

[Table 6]

| | | Foundation | Polymer particles | Composition (weight ratio) | MV (μm) | MV-MN (μm) | SD (μm) | Condition (12) | Condition (13) | Condition (14) |
|---|---|---|---|---|---|---|---|---|---|---|
| Example | 1 | 1 | A1+A2 | 1:1 | 10.9 | 5.8 | 3.7 | × | ○ | × |
| | 2 | 2 | A1+A4 | 1:1 | 8.4 | 4.7 | 4.6 | ○ | × | ○ |
| | 3 | 3 | A4+A5 | 1:1 | 7.4 | 3.5 | 4.3 | ○ | × | × |
| | 4 | 4 | A4+A6+B3 | 4:5:1 | 6.1 | 3.2 | 3.5 | ○ | ○ | ○ |
| | 5 | 5 | A2+A4+A6 | 3:3:4 | 7.5 | 4.7 | 5.2 | ○ | ○ | ○ |
| | 6 | 6 | A4+A5+A6 | 3:3:4 | 6.8 | 3.4 | 4.1 | ○ | ○ | ○ |
| | 7 | 7 | A1+A3+B1 | 4:3:3 | 12.1 | 5.0 | 8.1 | ○ | ○ | ○ |
| | 8 | 8 | A4+A5+B2 | 3:4:3 | 8.7 | 5.2 | 5.0 | ○ | ○ | ○ |
| Comparative Example | 1 | 9 | B3 | 100 | 5.0 | 1.4 | 1.3 | - | - | - |
| | 2 | 10 | B4 | 100 | 10.0 | 4.9 | 2.4 | - | - | - |

Eighteen persons were selected as panelists, and for the feeling on use and the difference before and after use of foundations 1 to 10, 6 items of "adhesiveness to skin", "fit on application", "feeling on use", "soft focus properties", "gloss/glossy feeling", and "sustainability of makeup effect (4 hours)" were comprehensively evaluated and each foundation was evaluated for the composition as a cosmetic with A to K below.

| | |
|---|---|
| A: | Foundation 1 is best. |
| B: | Foundation 2 is best. |
| C: | Foundation 3 is best. |
| D: | Foundation 4 is best. |
| E: | Foundation 5 is best. |
| F: | Foundation 6 is best. |
| G: | Foundation 7 is best. |
| H: | Foundation 8 is best. |
| I: | Foundation 9 is best. |
| J: | Foundation 10 is best. |
| K: | No difference between Foundations |

The evaluations of the panelists were as follows:

| | |
|---|---|
| A: | 1 panelist |
| B: | 2 panelists |
| C: | 1 panelist |
| D: | 2 panelists |
| E: | 3 panelists |
| F: | 3 panelists |
| G: | 3 panelists |
| H: | 3 panelists |
| I: | none |
| J: | none |
| K: | none. |

For the foundations 2, 3, 4, 5, 6, 7 and 8, there were many opinions that "adhesiveness to skin" and "sustainability of makeup effect (4 hours)" were particularly excellent. For the foundations 2, 3, 4, 5, 6 and 8, there were many opinions that "soft focus properties" was particularly excellent. For the foundations 1, 2, 5 and 7, there were many opinions that "gloss/glossy feeling" was particularly excellent. For "fit on application" and "feeling on use", though the panelists showed positive opinions for the foundations 1, 2, 3, 4, 5, 6, 7 and 8, opinions were somewhat divided depending on the preference of panelists. It is presumed that this means that in other words, spheroid particles are useful particles that enable the appropriate selection of prolate-spheroidal shapes and sizes according to the concept. Meanwhile, for the foundations 9 and 10, there were many opinions that "adhesiveness to skin", "sustainability of makeup effect (4 hours)", and "soft focus properties" were bad.

[Evaluation test 4] Powdery foundation

[Examples 9 to 16, Comparative Examples 3 to 4]

Makeup compositions (foundations 11 to 20) were produced using the polymer particles A1 to A6 and B1 to B4 according to the compositions described in Table 7 below.

The volume mean particle size (MV) and the dispersion range (SD) in the combinations of polymer particles compounded in foundations 11 to 20 were measured. The results are shown in Table 8.

[Table 7]

| | | Example | | | | | | | | Comparative Example | |
|---|---|---|---|---|---|---|---|---|---|---|---|
| | | 9 | 10 | 11 | 12 | 13 | 14 | 15 | 16 | 3 | 4 |
| | | Foundation | | | | | | | | | |
| | | 11 | 12 | 13 | 14 | 15 | 16 | 17 | 18 | 19 | 20 |
| Component (% by weight) | 2% alkyl acrylate/methacrylate copolymer dispersion | 15 | 15 | 15 | 15 | 15 | 15 | 15 | 15 | 15 | 15 |
| | 2% carboxy vinyl polymer dispersion | 15 | 15 | 15 | 15 | 15 | 15 | 15 | 15 | 15 | 15 |
| | Dipropylene glycol | 5 | 5 | 5 | 5 | 5 | 5 | 5 | 5 | 5 | 5 |
| | Edetate disodium | 0.05 | 0.05 | 0.05 | 0.05 | 0.05 | 0.05 | 0.05 | 0.05 | 0.05 | 0.05 |
| | Purified water | 2.7 | 2.7 | 2.7 | 2.7 | 2.7 | 2.7 | 2.7 | 2.7 | 2.7 | 2.7 |
| | Ethanol | 15 | 15 | 15 | 15 | 15 | 15 | 15 | 15 | 15 | 15 |
| | Sorbitan monoisostearate | 2 | 2 | 2 | 2 | 2 | 2 | 2 | 2 | 2 | 2 |
| | Polyoxyethylene (2) alkyl (12-16) ether phosphoric acid | 0.5 | 0.5 | 0.5 | 0.5 | 0.5 | 0.5 | 0.5 | 0.5 | 0.5 | 0.5 |
| | 2-ethylhexyl hydroxystearate | 5 | 5 | 5 | 5 | 5 | 5 | 5 | 5 | 5 | 5 |
| | Methylcyclopolysiloxane | 5 | 5 | 5 | 5 | 5 | 5 | 5 | 5 | 5 | 5 |
| | 2-amino-2-methyl-1-propanol | 0.5 | 0.5 | 0.5 | 0.5 | 0.5 | 0.5 | 0.5 | 0.5 | 0.5 | 0.5 |
| | Titanium oxide | 9.5 | 9.5 | 9.5 | 9.5 | 9.5 | 9.5 | 9.5 | 9.5 | 9.5 | 9.5 |
| | Red iron oxide | 0.2 | 0.2 | 0.2 | 0.2 | 0.2 | 0.2 | 0.2 | 0.2 | 0.2 | 0.2 |
| | Yellow iron oxide | 1 | 1 | 1 | 1 | 1 | 1 | 1 | 1 | 1 | 1 |
| | Black iron oxide | 0.1 | 0.1 | 0.1 | 0.1 | 0.1 | 0.1 | 0.1 | 0.1 | 0.1 | 0.1 |
| | Talc | 3.7 | 3.7 | 3.7 | 3.7 | 3.7 | 3.7 | 3.7 | 3.7 | 3.7 | 3.7 |
| | Crosslinked silicone powder | 1.5 | 1.5 | 1.5 | 1.5 | 1.5 | 1.5 | 1.5 | 1.5 | 1.5 | 1.5 |
| | 2% xanthan gum dispersion | 15 | 15 | 15 | 15 | 15 | 15 | 15 | 15 | 15 | 15 |
| | Phenoxyethanol | 0.2 | 0.2 | 0.2 | 0.2 | 0.2 | 0.2 | 0.2 | 0.2 | 0.2 | 0.2 |
| | Sodium dehydroacetate | 0.05 | 0.05 | 0.05 | 0.05 | 0.05 | 0.05 | 0.05 | 0.05 | 0.05 | 0.05 |
| | Polymer particles A1 | 1.5 | - | - | 0.5 | - | - | 0.5 | - | - | - |
| | Polymer particles A2 | 1.5 | - | 0.6 | - | - | - | - | - | - | - |
| | Polymer particles A3 | - | - | - | - | 0.5 | - | 0.5 | - | - | - |
| | Polymer particles A4 | - | 1.0 | 0.6 | 1.0 | - | - | - | 1.0 | - | - |
| | Polymer particles A5 | - | | 0.6 | 0.5 | 1.0 | 1.0 | 0.5 | | - | - |
| | Polymer particles A6 | - | 2.0 | 1.2 | 1.0 | 1.0 | 1.0 | 0.5 | 1.0 | - | - |
| | Polymer particles B1 | - | - | - | - | 0.5 | 1.0 | - | - | - | - |
| | Polymer particles B2 | - | - | - | - | - | - | 1.0 | 1.0 | - | - |
| | Polymer particles B3 | - | - | - | - | - | - | - | - | 3.0 | - |
| | Polymer particles B4 | - | - | - | - | - | - | - | - | - | 3.0 |

[Table 8]

| | | Foundation | Polymer particles | Composition (weight ratio) | MV (μm) | MV-MN (μm) | SD (μm) | Condition (12) | Condition (13) | Condition (14) |
|---|---|---|---|---|---|---|---|---|---|---|
| Example | 9 | 11 | A1+A2 | 1:1 | 10.9 | 5.8 | 3.7 | × | ○ | × |
| | 10 | 12 | A4+A6 | 1:2 | 5.7 | 3.1 | 3.3 | ○ | ○ | × |
| | 11 | 13 | A2+A4+A5+A6 | 1:1:1:2 | 7.0 | 4.0 | 4.4 | ○ | ○ | ○ |
| | 12 | 14 | A1+A4+A5+A6 | 1:2:1:2 | 6.9 | 3.7 | 4.0 | ○ | ○ | ○ |
| | 13 | 15 | A3+A5+A6+B1 | 1:2:2:1 | 11.1 | 6.2 | 6.6 | ○ | ○ | ○ |
| | 14 | 16 | A5+A6+B1 | 1:1:1 | 8.8 | 4.8 | 5.3 | ○ | ○ | ○ |
| | 15 | 17 | A1+A3+A5+A6+B2 | 1:1:1:1:2 | 10.3 | 5.1 | 5.1 | ○ | ○ | ○ |
| | 16 | 18 | A4+A6+B2 | 1:1:1 | 7.8 | 4.6 | 4.5 | ○ | ○ | ○ |
| Comparative Example | 3 | 19 | B3 | 100 | 5.0 | 1.4 | 1.3 | - | - | - |
| | 4 | 20 | B4 | 100 | 10.0 | 4.9 | 2.4 | - | - | - |

Eighteen persons were selected as panelists, and for the feeling on use and the difference before and after use for foundations 11 to 20, 6 items of "adhesiveness to skin", "fit on application", "feeling on use", "soft focus properties", "gloss/glossy feeling", and "sustainability of makeup effect (4 hours)" were comprehensively evaluated and each foundation was evaluated for the composition as a cosmetic with A to K below.

A: Foundation 11 is best.
B: Foundation 12 is best.
C: Foundation 13 is best.
D: Foundation 14 is best.
E: Foundation 15 is best.
F: Foundation 16 is best.
G: Foundation 17 is best.
H: Foundation 18 is best.
I: Foundation 19 is best.
J: Foundation 20 is best.
K: No difference between Foundations

The evaluations of the panelists were as follows:

A: 1 panelist
B: 2 panelists
C: 2 panelists
D: 3 panelists
E: 2 panelists
F: 3 panelists
G: 2 panelists
H: 3 panelists
I: none
J: none
K: none.

For the foundations 10 to 15, there were many opinions that "adhesiveness to skin" and "sustainability of makeup effect (4 hours)" were particularly excellent, and "soft focus properties" was also good. In particular, for "fit on application" and "feeling on use", opinions were somewhat divided depending on the preference of panelists for foundations 11 to 18. There was an opinion that particles with a relatively high aspect ratio had high impact as feeling, and the larger the particle size of the particles added, the better the gloss/glossy feeling. Meanwhile, for the foundations 19 and 20, there were many opinions that "adhesiveness to skin", "sustainability of makeup effect (4 hours)", and "soft focus properties" were bad.

As described above, the skin cosmetic including the prolate-spheroidal polymer particles A has high adhesion, and useful unique properties in terms of light diffusibility (including protection against UV), feelings, fluidity and the like while maximizing the weight reduction and transparency of the polymer component. In particular, by mixing particles having different sizes and shapes, and further, appropriately mixing particles having a flat ellipsoidal shape, multiple synergistic effects can be expected, and the particles can be used effectively as a skin cosmetic.

[4] Production of various skin cosmetics

[Formulation Examples 1 to 30]

Because cosmetics in general, especially skin cosmetics, have many uses and products, we examined based on cosmetic formulations generally disclosed whether the prolate-spheroidal polymer particles A can be applied as skin cosmetics. As a result, it was confirmed that the prolate-spheroidal polymer particles A can be handled in the same manner as in existing polymer particles, inorganic particles and the like, and that the peculiar properties of the prolate-spheroidal polymer particles A can be added.

We examined whether the prolate-spheroidal polymer particles A can be applied as cosmetics, and as a result, it was confirmed that the prolate-spheroidal polymer particles A can be handled in the same manner as in existing polymer powders, inorganic powders and the like, and that the peculiar properties of the shapes can be added. The properties that are superior are also different depending on the shape, the compounding amount, and the compounding ratio, and thus, it was confirmed that by appropriately selecting and adjusting the type of the shape, the compounding amount, the component used and the like according to the purpose and concept of the product, the particles can be applied to many cosmetic uses, and the application is easy for those skilled in the art from the technical point of view.

Formulation examples of skin cosmetics including prolate-spheroidal polymer particles are described below. Various skin cosmetics were produced using the polymer particles A1 to A7 according to the compositions shown in Tables 9 to 13 below. Numerical values in Tables show the content (% by weight) of each component.

(1) Powder cosmetic

[Table 9]

| Powder foundation | | Formulation Example | | | | | |
|---|---|---|---|---|---|---|---|
| | | 1 | 2 | 3 | 4 | 5 | 6 |
| Component (% by weight) | Perfluorooctyltriethoxysilane-treated red iron oxide | 1.0 | 1.0 | 1.0 | 1.0 | 1.0 | 1.0 |
| | Perfluorooctyltriethoxysilane-treated yellow iron oxide | 4.0 | 4.0 | 4.0 | 4.0 | 4.0 | 4.0 |
| | Perfluorooctyltriethoxysilane-treated black iron oxide | 0.5 | 0.5 | 0.5 | 0.5 | 0.5 | 0.5 |
| | Perfluorooctyltriethoxysilane-treated titanium oxide | 10.0 | 10.0 | 10.0 | 10.0 | 10.0 | 10.0 |
| | Silicone treated strong cohesive titanium oxide | 8.0 | 8.0 | 8.0 | 8.0 | 8.0 | 8.0 |
| | Aluminum hydroxide/N-lauroyl lysine-treated needle-like titanium oxide | 3.0 | 3.0 | 3.0 | 3.0 | 3.0 | 3.0 |
| | Silicone treated talc | 21.3 | 21.3 | 21.3 | 21.3 | 21.3 | 21.3 |
| | Silicone treated talc silicon potassium fluoride calcined product | 5.0 | 5.0 | 5.0 | 5.0 | 5.0 | 5.0 |
| | Boron nitride | 10.0 | 10.0 | 10.0 | 10.0 | 10.0 | 10.0 |
| | Perfluoroalkyl phosphate diethanol amine-treated mica | 5.0 | 5.0 | 5.0 | 5.0 | 5.0 | 5.0 |
| | Zinc myristate-treated zinc oxide/titanium oxide coated mica | 10.0 | 10.0 | 10.0 | 10.0 | 10.0 | 10.0 |
| | Crosslinked silicone powder | 0.6 | 0.6 | 0.6 | 0.6 | 0.6 | 0.6 |
| | Sodium hyaluronate-treated silk powder | 1.0 | 1.0 | 1.0 | 1.0 | 1.0 | 1.0 |
| | Chlorphenesin | 0.2 | 0.2 | 0.2 | 0.2 | 0.2 | 0.2 |
| | Sodium dehydroacetate | 0.2 | 0.2 | 0.2 | 0.2 | 0.2 | 0.2 |
| | Isostearic acid hydrogenated castor oil | 1.0 | 1.0 | 1.0 | 1.0 | 1.0 | 1.0 |
| | Methylpolysiloxane | 4.0 | 4.0 | 4.0 | 4.0 | 4.0 | 4.0 |
| | Isocetyl myristate | 1.0 | 1.0 | 1.0 | 1.0 | 1.0 | 1.0 |
| | 2-ethylhexyl paramethoxy cinnamate | 2.0 | 2.0 | 2.0 | 2.0 | 2.0 | 2.0 |
| | Glyceryl tri(caprylate/caprate) | 2.0 | 2.0 | 2.0 | 2.0 | 2.0 | 2.0 |
| | Water soluble collagen solution | 0.1 | 0.1 | 0.1 | 0.1 | 0.1 | 0.1 |
| | Citrus unshiu peel extract | 0.1 | 0.1 | 0.1 | 0.1 | 0.1 | 0.1 |
| | Spherical methyl polymethacrylate | 5.0 | 5.0 | 5.0 | 5.0 | 5.0 | 5.0 |
| | Polymer particles A1 | 3.0 | - | 1.0 | 1.0 | - | - |
| | Polymer particles A2 | - | 4.0 | - | 2.0 | - | - |
| | Polymer particles A3 | - | - | 1.0 | 2.0 | - | - |
| | Polymer particles A4 | 1.0 | - | - | - | 3.0 | 2.0 |
| | Polymer particles A5 | - | - | 1.5 | - | - | 2.0 |
| | Polymer particles A6 | - | 1.0 | - | - | 2.0 | 1.0 |
| | Polymer particles A7 | 1.0 | - | 1.5 | - | - | - |

(2) Oil cosmetic

[Table 10]

| Lipstick | | Formulation Example | | | | | |
|---|---|---|---|---|---|---|---|
| | | 7 | 8 | 9 | 10 | 11 | 12 |
| Component (% by weight) | Paraffin wax | 3.0 | 3.0 | 3.0 | 3.0 | 3.0 | 3.0 |
| | Synthetic hydrocarbon wax | 3.0 | 3.0 | 3.0 | 3.0 | 3.0 | 3.0 |
| | Ethylene propylene copolymer | 3.0 | 3.0 | 3.0 | 3.0 | 3.0 | 3.0 |
| | Ceresin | 3.0 | 3.0 | 3.0 | 3.0 | 3.0 | 3.0 |
| | Microcrystalline wax | 3.0 | 3.0 | 3.0 | 3.0 | 3.0 | 3.0 |
| | Vaseline | 5.0 | 5.0 | 5.0 | 5.0 | 5.0 | 5.0 |
| | Heavy liquid isoparaffin | 25.0 | 25.0 | 25.0 | 25.0 | 25.0 | 25.0 |
| | Di(octyldodecyl/phytosteryl/behenil) lauroyl glutamate | 10.0 | 10.0 | 10.0 | 10.0 | 10.0 | 10.0 |
| | Polyglyceryl triisostearate | 5.0 | 5.0 | 5.0 | 5.0 | 5.0 | 5.0 |
| | Octyl dodecanol | 5.0 | 5.0 | 5.0 | 5.0 | 5.0 | 5.0 |
| | Squalane | 5.0 | 5.0 | 5.0 | 5.0 | 5.0 | 5.0 |
| | Hydrogenated polydecene | 5.0 | 5.0 | 5.0 | 5.0 | 5.0 | 5.0 |
| | Glycerin tri(caprylate/caprate) | 13.6 | 13.6 | 13.6 | 13.6 | 13.6 | 13.6 |
| | Tocopherol | 0.1 | 0.1 | 0.1 | 0.1 | 0.1 | 0.1 |
| | Red No. 201 | 0.1 | 0.1 | 0.1 | 0.1 | 0.1 | 0.1 |
| | Red No. 202 | 0.1 | 0.1 | 0.1 | 0.1 | 0.1 | 0.1 |
| | Needle-like titanium oxide | 1.0 | 1.0 | 1.0 | 1.0 | 1.0 | 1.0 |
| | Iron oxide | 0.5 | 0.5 | 0.5 | 0.5 | 0.5 | 0.5 |
| | Silica | 3.0 | 3.0 | 3.0 | 3.0 | 3.0 | 3.0 |
| | Mica | 2.0 | 2.0 | 2.0 | 2.0 | 2.0 | 2.0 |
| | Mica titanium | 1.5 | 1.5 | 1.5 | 1.5 | 1.5 | 1.5 |
| | Titanium oxide coated glass flakes | 2.0 | 2.0 | 2.0 | 2.0 | 2.0 | 2.0 |
| | Raspberry extract | 0.1 | 0.1 | 0.1 | 0.1 | 0.1 | 0.1 |
| | Polymer particles A1 | 1.0 | - | - | - | - | - |
| | Polymer particles A2 | - | 1.0 | - | - | - | - |
| | Polymer particles A3 | - | - | - | - | - | - |
| | Polymer particles A4 | - | - | 0.5 | 1.0 | 0.5 | - |
| | Polymer particles A5 | - | - | - | - | - | 1.0 |
| | Polymer particles A6 | - | - | - | - | 0.5 | - |
| | Polymer particles A7 | - | - | 0.5 | - | - | - |

(3) Oil-in-water emulsified cosmetic

[Table 11]

| Mascara | | Formulation Example | | | | | |
|---|---|---|---|---|---|---|---|
| | | 13 | 14 | 15 | 16 | 17 | 18 |
| Component (% by weight) | Purified water | 43.9 | 43.9 | 43.9 | 43.9 | 43.9 | 43.9 |
| | Bentonite | 1.5 | 1.5 | 1.5 | 1.5 | 1.5 | 1.5 |
| | Phenoxyethanol | 0.5 | 0.5 | 0.5 | 0.5 | 0.5 | 0.5 |
| | Hydroxyethyl cellulose | 2.0 | 2.0 | 2.0 | 2.0 | 2.0 | 2.0 |
| | Sericite | 0.5 | 0.5 | 0.5 | 0.5 | 0.5 | 0.5 |
| | PEG-20 glyceryl stearate | 5.0 | 5.0 | 5.0 | 5.0 | 5.0 | 5.0 |
| | Butylene glycol | 6.0 | 6.0 | 6.0 | 6.0 | 6.0 | 6.0 |
| | Boron nitride | 12.0 | 12.0 | 12.0 | 12.0 | 12.0 | 12.0 |
| | Iron oxide | 2.0 | 2.0 | 2.0 | 2.0 | 2.0 | 2.0 |
| | Titanium oxide | 1.0 | 1.0 | 1.0 | 1.0 | 1.0 | 1.0 |
| | Triethanolamine | 1.0 | 1.0 | 1.0 | 1.0 | 1.0 | 1.0 |
| | Carnauba wax | 1.0 | 1.0 | 1.0 | 1.0 | 1.0 | 1.0 |
| | Bees wax | 6.5 | 6.5 | 6.5 | 6.5 | 6.5 | 6.5 |
| | Cetanol | 2.0 | 2.0 | 2.0 | 2.0 | 2.0 | 2.0 |
| | Stearic acid | 2.0 | 2.0 | 2.0 | 2.0 | 2.0 | 2.0 |
| | Polyisobutene | 1.0 | 1.0 | 1.0 | 1.0 | 1.0 | 1.0 |
| | Tocopheryl acetate | 0.1 | 0.1 | 0.1 | 0.1 | 0.1 | 0.1 |
| | Acrylates copolymer | 9.0 | 9.0 | 9.0 | 9.0 | 9.0 | 9.0 |
| | Polymer particles A1 | - | - | - | 0.5 | - | 0.5 |
| | Polymer particles A2 | - | 2.0 | - | - | - | - |
| | Polymer particles A3 | - | - | 0.5 | - | - | - |
| | Polymer particles A4 | - | - | 0.5 | 1.0 | 1.0 | 1.0 |
| | Polymer particles A5 | - | 0.5 | - | - | - | 1.0 |
| | Polymer particles A6 | 3.0 | 0.5 | 2.0 | 1.0 | 2.0 | - |
| | Polymer particles A7 | - | - | - | 0.5 | - | 0.5 |

(4) Water-in-oil emulsified cosmetic and others

[Table 12]

| Water-in-oil emulsified foundation | | Formulation Example 19 | 20 | 21 | 22 | 23 | 24 |
|---|---|---|---|---|---|---|---|
| Component (% by weight) | Diglyceryl monoisostearate | 1.0 | 1.0 | 1.0 | 1.0 | 1.0 | 1.0 |
| | Polyether modified silicone mixture | 5.0 | 5.0 | 5.0 | 5.0 | 5.0 | 5.0 |
| | Volatile silicone | 15.0 | 15.0 | 15.0 | 15.0 | 15.0 | 15.0 |
| | Isododecane | 5.0 | 5.0 | 5.0 | 5.0 | 5.0 | 5.0 |
| | Dipentaerythrityl tripolyhydroxystearate | 3.0 | 3.0 | 3.0 | 3.0 | 3.0 | 3.0 |
| | Liquid paraffin | 6.0 | 6.0 | 6.0 | 6.0 | 6.0 | 6.0 |
| | Soy lecithin | 0.1 | 0.1 | 0.1 | 0.1 | 0.1 | 0.1 |
| | Propylene glycol dicaprylate | 4.0 | 4.0 | 4.0 | 4.0 | 4.0 | 4.0 |
| | Glycerin | 3.0 | 3.0 | 3.0 | 3.0 | 3.0 | 3.0 |
| | Maltitol | 3.0 | 3.0 | 3.0 | 3.0 | 3.0 | 3.0 |
| | Ginseng extract | 0.1 | 0.1 | 0.1 | 0.1 | 0.1 | 0.1 |
| | Sambucus nigra flower extract | 0.1 | 0.1 | 0.1 | 0.1 | 0.1 | 0.1 |
| | Octyltriethoxysilane-treated titanium oxide | 10.0 | 10.0 | 10.0 | 10.0 | 10.0 | 10.0 |
| | Octyltriethoxysilane-treated red iron oxide | 0.2 | 0.2 | 0.2 | 0.2 | 0.2 | 0.2 |
| | Octyltriethoxysilane-treated yellow iron oxide | 0.8 | 0.8 | 0.8 | 0.8 | 0.8 | 0.8 |
| | Octyltriethoxysilane-treated black iron oxide | 0.1 | 0.1 | 0.1 | 0.1 | 0.1 | 0.1 |
| | Talc | 0.9 | 0.9 | 0.9 | 0.9 | 0.9 | 0.9 |
| | Purified water | 40.85 | 40.85 | 40.85 | 40.85 | 40.85 | 40.85 |
| | Sodium dehydroacetate | 0.05 | 0.05 | 0.05 | 0.05 | 0.05 | 0.05 |
| | Phenoxyethanol | 0.3 | 0.3 | 0.3 | 0.3 | 0.3 | 0.3 |
| | Crosslinked silicone powder | 0.5 | 0.5 | 0.5 | 0.5 | 0.5 | 0.5 |
| | Polymer particles A1 | 1.0 | - | - | - | - | - |
| | Polymer particles A2 | - | 1.0 | - | - | - | - |
| | Polymer particles A3 | - | - | 0.5 | - | - | - |
| | Polymer particles A4 | - | - | - | 1.0 | - | - |
| | Polymer particles A5 | - | - | - | - | 0.5 | - |
| | Polymer particles A6 | - | - | 0.5 | - | - | 1.0 |
| | Polymer particles A7 | - | - | - | - | 0.5 | - |

[Table 13]

| Sunscreen | | Formulation Example | | | | | |
|---|---|---|---|---|---|---|---|
| | | 25 | 26 | 27 | 28 | 29 | 30 |
| Component (% by weight) | Sorbitan monoisostearate | 1.0 | 1.0 | 1.0 | 1.0 | 1.0 | 1.0 |
| | Polyether modified silicone mixture | 5.0 | 5.0 | 5.0 | 5.0 | 5.0 | 5.0 |
| | Volatile silicone | 15.0 | 15.0 | 15.0 | 15.0 | 15.0 | 15.0 |
| | Isohexadecane | 5.0 | 5.0 | 5.0 | 5.0 | 5.0 | 5.0 |
| | Di(phytosteryl/isostearyl/cetyl/stearyl/behenil) dimer dilinoleate | 0.2 | 0.2 | 0.2 | 0.2 | 0.2 | 0.2 |
| | Octyl cinnamate | 5.0 | 5.0 | 5.0 | 5.0 | 5.0 | 5.0 |
| | Diethylaminohydroxybenzoyl Hexyl benzoate | 1.0 | 1.0 | 1.0 | 1.0 | 1.0 | 1.0 |
| | Squalane | 3.0 | 3.0 | 3.0 | 3.0 | 3.0 | 3.0 |
| | Purified water | 43.6 | 43.6 | 43.6 | 43.6 | 43.6 | 43.6 |
| | Ethanol | 10.0 | 10.0 | 10.0 | 10.0 | 10.0 | 10.0 |
| | Fine particles titanium oxide | 3.0 | 3.0 | 3.0 | 3.0 | 3.0 | 3.0 |
| | Fine particles zinc oxide | 3.0 | 3.0 | 3.0 | 3.0 | 3.0 | 3.0 |
| | Titanium oxide | 1.0 | 1.0 | 1.0 | 1.0 | 1.0 | 1.0 |
| | Trimethylsiloxy silicic acid | 3.0 | 3.0 | 3.0 | 3.0 | 3.0 | 3.0 |
| | Phenoxyethanol | 0.2 | 0.2 | 0.2 | 0.2 | 0.2 | 0.2 |
| | Polymer particles A1 | 1.0 | - | - | - | - | - |
| | Polymer particles A2 | - | 1.0 | - | - | - | |
| | Polymer particles A3 | - | - | 0.3 | - | - | - |
| | Polymer particles A4 | - | - | - | 1.0 | - | - |
| | Polymer particles A5 | - | - | - | - | 1.0 | - |
| | Polymer particles A6 | - | - | 0.5 | - | - | 1.0 |
| | Polymer particles A7 | - | - | 0.2 | - | - | - |

## Claims

**1.** A skin cosmetic comprising:

(A) 0.1 to 40% by weight of polymer particles including prolate-spheroidal polymer particles A that satisfy (1) to (3) below:

(1) an average ($L^1_{AV}$) of a long diameter ($L^1$) of a two-dimensional projection obtained by radiating light from a direction orthogonal to a major axis direction of 3 to 60 μm;
(2) an average ($D^1_{AV}$) of a short diameter ($D^1$) of a two-dimensional projection obtained by radiating light from a direction orthogonal to a major axis direction of 0.5 to 30 μm; and
(3) an average ($P^1_{AV}$) of an aspect ratio ($L^1/D^1$) calculated from the long diameter ($L^1$) and the short diameter ($D^1$) of 1.5 to 30,

and have no flat surface; and
60 to 99.9% by weight of other components including (B) inorganic particles and (C) an oily component.

**2.** The skin cosmetic according to claim 1, wherein the prolate-spheroidal polymer particles A further satisfy (4):

(4) a softening temperature of 120 to 180°C.

**3.** The skin cosmetic according to claims 1 or 2, wherein the prolate-spheroidal polymer particles A have, at least on a surface or in a surface layer of the prolate-spheroidal polymer particles A, fine irregularities formed by binding or containing fine particles that are composed of a component same as a component of the prolate-spheroidal polymer particles A and satisfy (5) below:
(5) a particle size ($SP^1$) of the fine particles which are attached or contained on the surface or in the surface layer is $1/1000 \times D^1_{AV} \leq SP^1 \leq 1/2 \times D^1_{AV}$.

**4.** The skin cosmetic according to any one of claims 1 to 3, wherein the prolate-spheroidal polymer particles A include a styrene polymer or a (meth)acrylic polymer.

**5.** The skin cosmetic according to any one of claims 1 to 4, wherein the prolate-spheroidal polymer particles A have a bulk density of 0.1 to 0.7 g/mL.

**6.** The skin cosmetic according to any one of claims 1 to 5, wherein the prolate-spheroidal polymer particles A have a volume mean particle size ($MV^1$) of 0.1 to 50 $\mu$m and satisfy a dispersion range ($SD^1$) of 1 to 30.

**7.** The skin cosmetic according to any one of claims 1 to 6, wherein the polymer particles (A) include two or more types of the prolate-spheroidal polymer particles A.

**8.** The skin cosmetic according to any one of claims 1 to 7, wherein the polymer particles (A) further include one or more types of polymer particles B having a shape different from a shape of the prolate-spheroidal polymer particles A.

**9.** The skin cosmetic according to claim 8, wherein the polymer particles B are flat ellipsoidal polymer particles that have a front view, a plan view, and a side view of projections according to third angle projection that are all ellipses, and that satisfy (6) to (8) below:

(6) an average ($L^2_{AV}$) of a long diameter ($L^2$) of a flat part of $0.13 \leq L^2_{AV} \leq 500$ $\mu$m;
(7) an average ($D^2_{AV}$) of a short diameter ($D^2$) of a flat part of $0.1 \leq D^2_{AV} \leq 250$ $\mu$m; and
(8) an average ($P^2_{AV}$) of an aspect ratio ($L^2/D^2$) calculated from the long diameter ($L^2$) and the short diameter ($D^2$) of $1.3 < P^2_{AV} \leq 50$.

**10.** The skin cosmetic according to claims 8 or 9, having a compounding ratio of the prolate-spheroidal polymer particles A to the polymer particles B of 99:1 to 10:90 in terms of weight ratio.

**11.** The skin cosmetic according to any one of claims 7 to 10, wherein the polymer particles (A) are polymer particles in which two or more types of polymer particles are combined so that the polymer particles in which two or more types of polymer particles are combined satisfy at least one of (12), (13), and (14) below:

(12) a difference between a maximum and a minimum of an average of an aspect ratio of two or more;
(13) a difference between a maximum and a minimum of a volume mean particle size of 3 $\mu$m or more; and
(14) appearance of two or more peaks in particle size distribution measurement.

**12.** The skin cosmetic according to any one of claims 7 to 11, wherein the polymer particles in which two or more types of polymer particles are combined have a difference between a volume mean particle size (MV) and a number mean particle size (MN) of 1.5 $\mu$m or more.

**13.** The skin cosmetic according to any one of claims 1 to 11, wherein the inorganic particles (B) have a plate-like shape or a scaly shape.

**14.** The skin cosmetic according to any one of claims 1 to 12, wherein the oily component (C) is at least one selected from a higher alcohol, a higher fatty acid, a fat and oil, a wax, a hydrocarbon, a silicone, and a fatty acid ester.

FIG.1

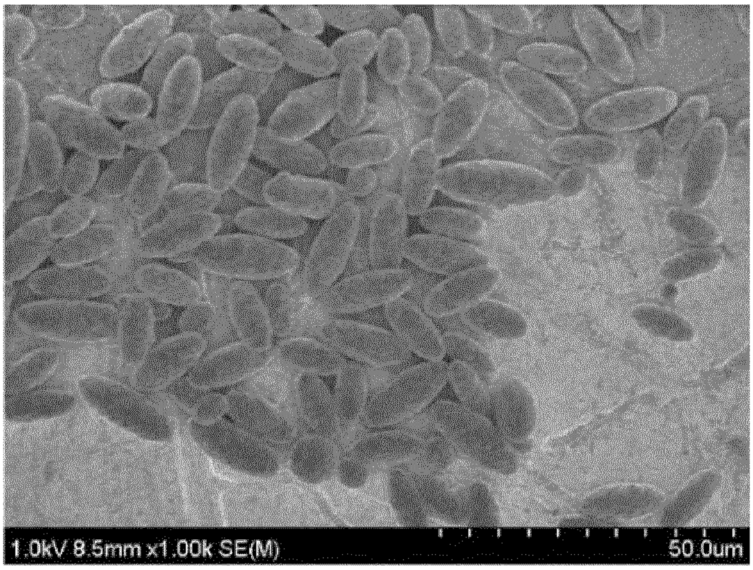


FIG.2

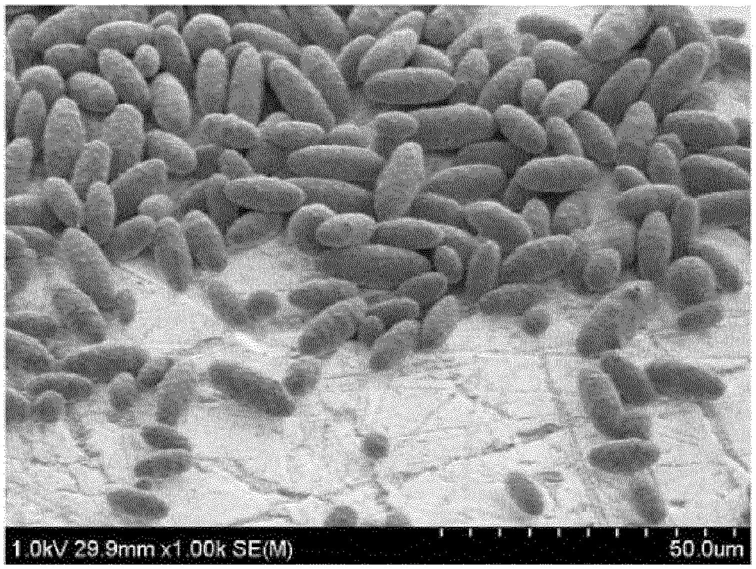

FIG.3

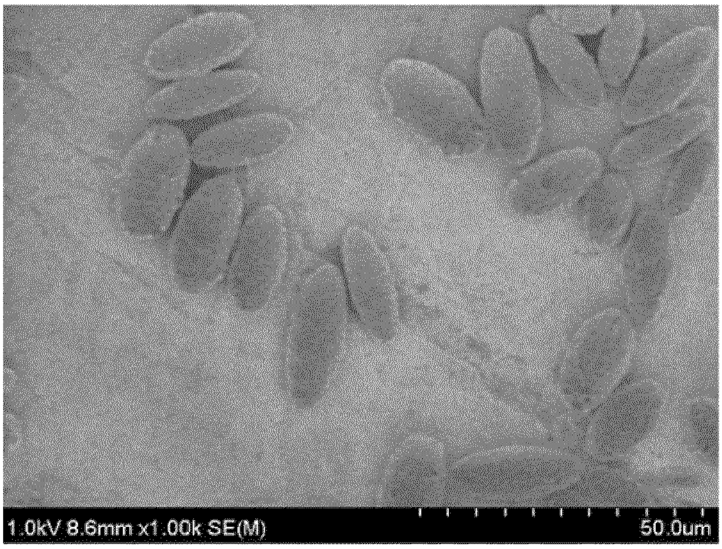


FIG.4

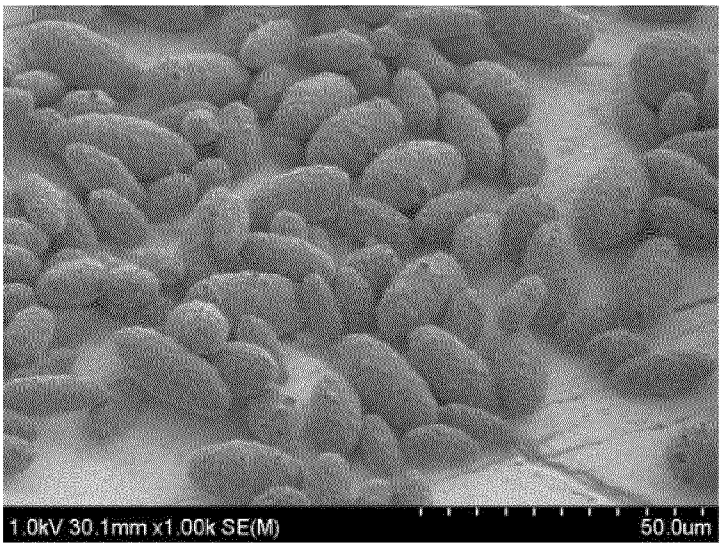

# FIG.5

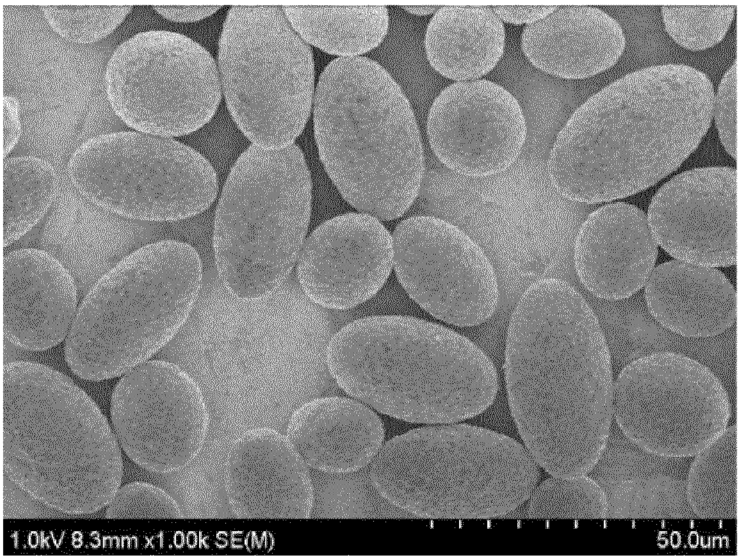


# FIG.6

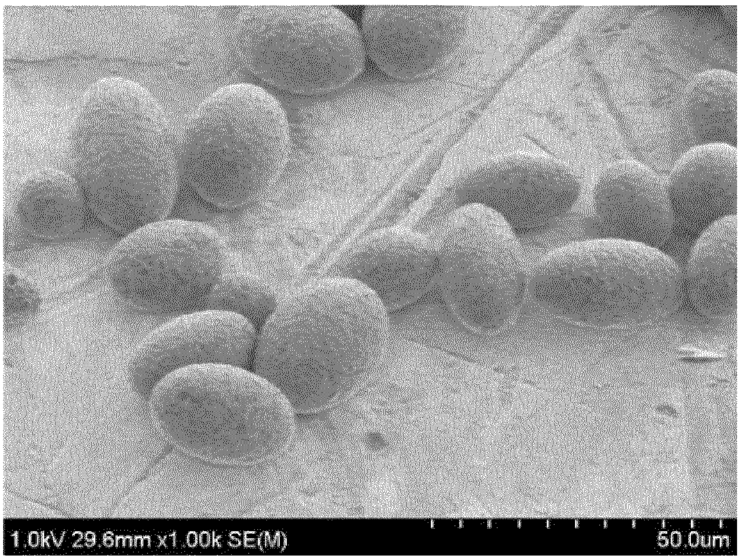

FIG.7

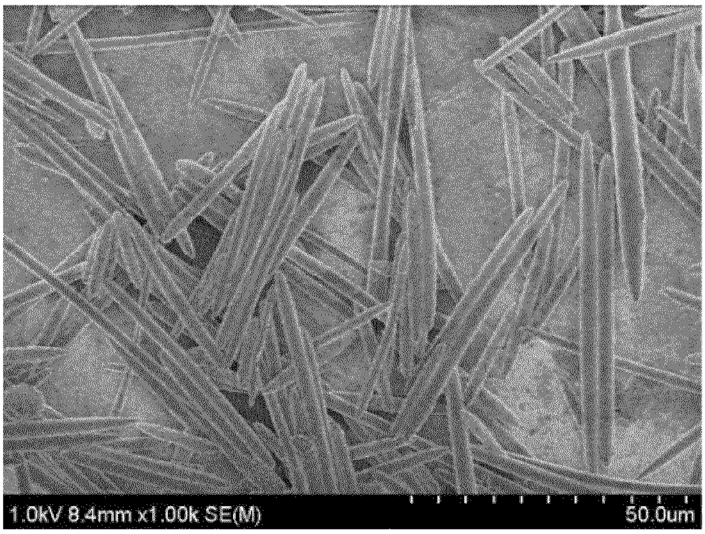


FIG.8

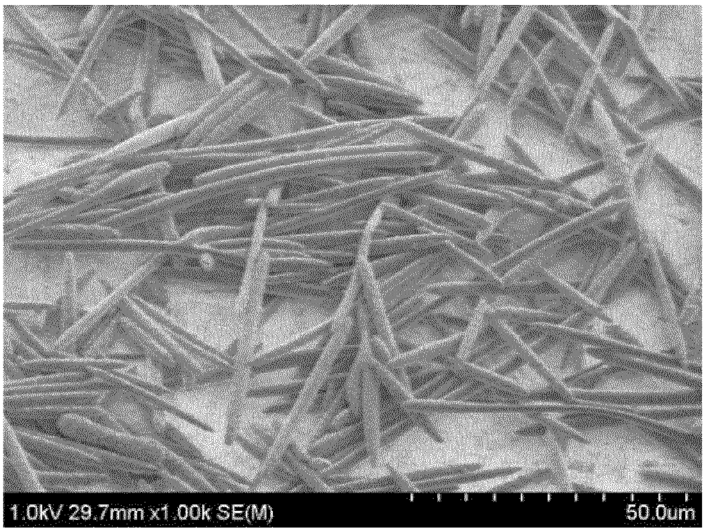

FIG.9

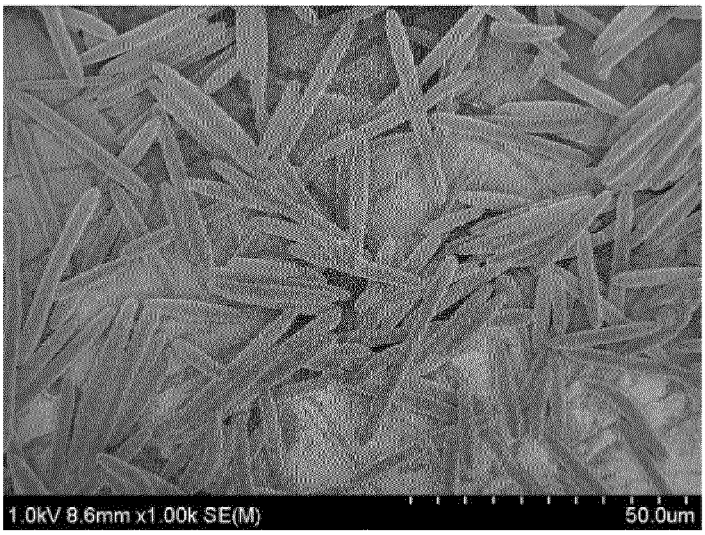


FIG.10

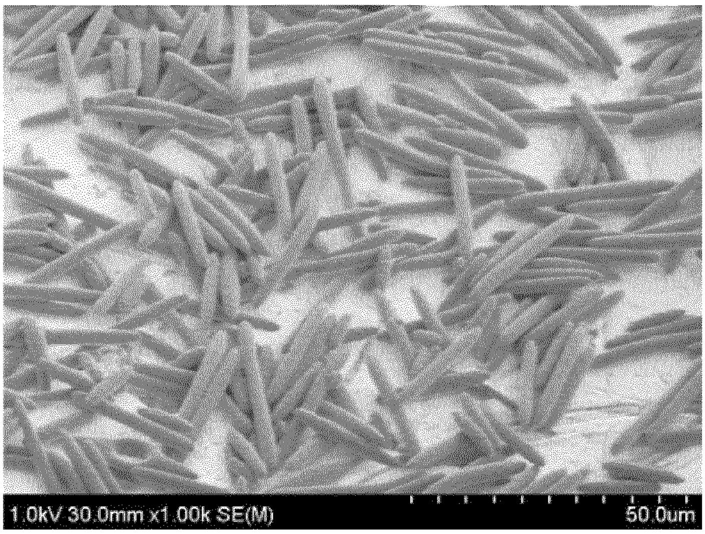

FIG.11

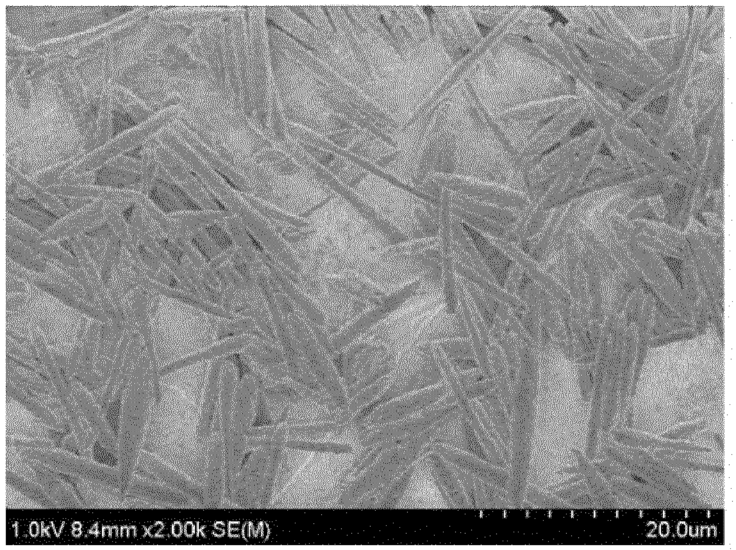


FIG.12

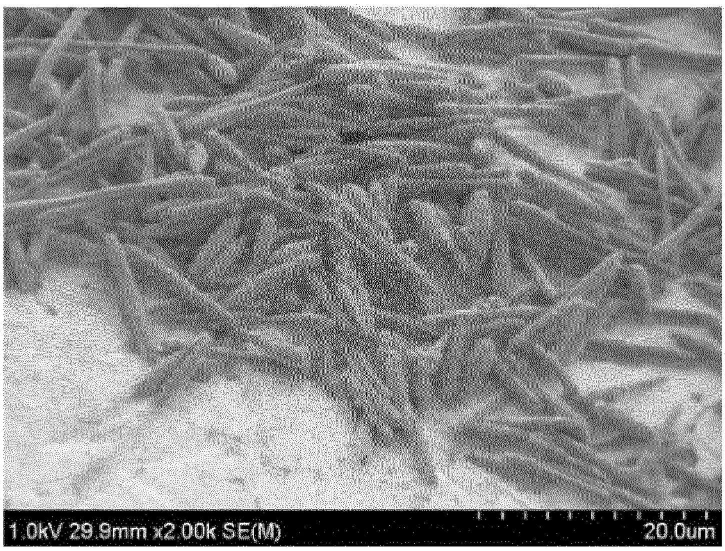

# FIG.13

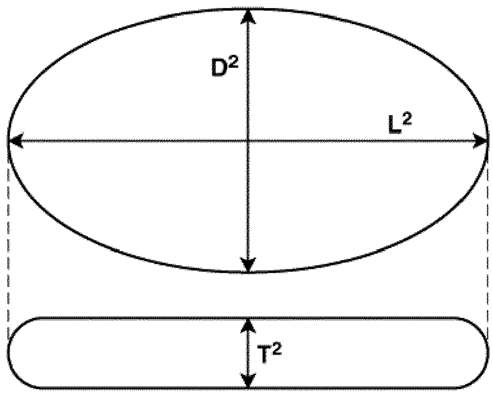

**INTERNATIONAL SEARCH REPORT**

International application No.
PCT/JP2018/006126

A. CLASSIFICATION OF SUBJECT MATTER
Int.Cl. A61K8/81(2006.01)i, A61K8/19(2006.01)i, A61K8/31(2006.01)i, A61K8/34(2006.01)i, A61K8/36(2006.01)i, A61K8/37(2006.01)i, A61K8/891(2006.01)i, A61K8/92(2006.01)i, A61Q1/00(2006.01)i

B. FIELDS SEARCHED
Minimum documentation searched (classification system followed by classification symbols)
Int.Cl. A61K8/81, A61K8/19, A61K8/31, A61K8/34, A61K8/36, A61K8/37, A61K8/891, A61K8/92, A61Q1/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2015-93973 A (NISSHINBO HOLDINGS INC.) 18 May 2015, claims, paragraphs [0039]-[0046], [0127], examples & US 2016/0262988 A1, claims, paragraphs [0050]-[0055], [0146], examples & WO 2015/072443 A1 & EP 3070138 A1 & CN 105722940 A | 1-8,10-14<br>9-14 |
| Y | WO 2016/181877 A1 (NISSHINBO HOLDINGS INC.) 17 November 2016, claims, paragraphs [0013], [0015], [0017], [0034], [0079], examples & JP 2017-171939 A & CN 107531811 A | 9-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 May 2018 (18.05.2018) | 29 May 2018 (29.05.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/006126

C (Continuation). DOCUMENTS CONSIDERED TO BE RELEVANT

| Category* | Citation of document, with indication, where appropriate, of the relevant passages | Relevant to claim No. |
|---|---|---|
| A | JP 2009-235355 A (NISSHINBO HOLDINGS INC.) 15 October 2009, claims, examples (Family: none) | 1-14 |
| A | WO 2016/181878 A1 (NISSHINBO HOLDINGS INC.) 17 November 2016 & US 2018/0104160 A1 & EP 3296327 A1 & CN 107614542 A | 1-14 |
| A | JP 2007-70372 A (NISSHINBO INDUSTRIES, INC.) 22 March 2007 & US 2007/0054123 A1 & KR 10-2007-0026122 A & CN 1927900 A & TW 200722439 A | 1-14 |
| A | JP 10-506646 A (L'OREAL) 30 June 1998 & US 5763500 A & WO 1996/038129 A1 & EP 745379 A1 & DE 69600043 T2 & FR 2734831 A1 & BR 9608684 A & CN 1185732 A | 1-14 |
| A | JP 5-201826 A (L'OREAL) 10 August 1993 & US 5223559 A & EP 502769 A1 & DE 69209877 T2 & FR 2673372 A1 & CA 2061952 A1 | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009235353 A
- JP 2009235355 A
- JP 2015093973 A
- WO 2016181876 A
- WO 2016181878 A
- WO 2016181877 A